(19) Europäisches Patentamt
European Patent Office
Office européen des brevets

(11) EP 2 985 284 B1

(12) EUROPEAN PATENT SPECIFICATION

(45) Date of publication and mention of the grant of the patent:
**03.11.2021 Bulletin 2021/44**

(21) Application number: **14782759.6**

(22) Date of filing: **10.04.2014**

(51) Int Cl.:
*C07D 403/12* (2006.01) *C07D 417/12* (2006.01)
*A61K 31/4178* (2006.01) *A61K 31/4184* (2006.01)
*A61K 31/427* (2006.01) *A61K 31/428* (2006.01)
*A61P 1/16* (2006.01) *A61P 3/10* (2006.01)
*A61P 7/06* (2006.01) *A61P 9/00* (2006.01)
*A61P 25/28* (2006.01) *A61P 25/32* (2006.01)
*A61P 31/00* (2006.01) *A61P 31/12* (2006.01)
*A61P 35/00* (2006.01) *A61P 39/06* (2006.01)
*A61P 43/00* (2006.01)

(86) International application number:
**PCT/RU2014/000265**

(87) International publication number:
**WO 2014/168523 (16.10.2014 Gazette 2014/42)**

(54) **DICARBOXYLIC ACID BISAMIDE DERIVATIVES, USE THEREOF, PHARMACEUTICAL COMPOSITION BASED THEREON AND METHODS FOR PRODUCING DICARBOXYLIC ACID BISAMIDE DERIVATIVES**

DICARBONSÄURE-BISAMID-DERIVATE, VERWENDUNG DAVON, DARAUF BASIERENDE PHARMAZEUTISCHE ZUSAMMENSETZUNG UND VERFAHREN ZUR HERSTELLUNG VON DICARBONSÄURE-BISAMID-DERIVATEN

DÉRIVÉS DE BISAMIDES D'ACIDES DICARBONIQUES, LEUR UTILISATION, COMPOSITION PHARMACEUTIQUE SUR LEUR BASE ET PROCÉDÉS DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.04.2013 RU 2013116822**

(43) Date of publication of application:
**17.02.2016 Bulletin 2016/07**

(73) Proprietor: **Treamid Therapeutics GmbH**
**13353 Berlin (DE)**

(72) Inventors:
- **NEBOLSIN, Vladimir Evgenievich**
**Moskovskaya obl., 143581 (RU)**
- **KROMOVA, Tatyana Alexandrovna**
**248010 Kaluga (RU)**
- **ZHELTUKHINA, Galina Alexandrovna**
**129344 Moscow (RU)**

(74) Representative: **Schulz Junghans Patentanwälte PartGmbB et al**
**Großbeerenstraße 71**
**10963 Berlin (DE)**

(56) References cited:
**JP-A- S6 169 759 RU-C2- 2 335 495**

- **CHARLET-FAGNERE C ET AL: "Syntheses of Large-Ring Bis-Indolic Dilactams", TETRAHEDRON LETTERS, PERGAMON, GB, vol. 40, no. 9, 26 February 1999 (1999-02-26), pages 1685-1688, XP004157166, ISSN: 0040-4039, DOI: 10.1016/S0040-4039(99)00065-9**
- **JIANG WEI-QUN ET AL: "Cytotoxic Bis-3,4-dihydro-.beta.-carbolines and Bis-.beta.-carbolines", JOURNAL OF ENZYME INHIBITION AND MEDICINAL CHEMISTRY, INFORMA HEALTHCARE, GB, vol. 17, no. 6, 1 January 2002 (2002-01-01), pages 369-374, XP009096614, ISSN: 1475-6366, DOI: 10.1080/1475630021000005631**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 985 284 B1

- "Synthesis of demethoxy analogs of melatonin from tryptamide and dicarboxylic acids", SOOBSCENIJA AKADEMII NAUK GRUZINSKOJ SSR - BULLETIN OF THEACADEMY OF SCIENCES OF THE GEORGIAN SSR, TBILISI, GE, vol. 115, 1 January 1984 (1984-01-01), pages 89-92, XP009192251, ISSN: 0132-1447
- BURAKOWSKI, TADEUSZ JULIUSZ ET AL.: 'Preparation of certain bis-compounds derived from beta-(2-thienyl)ethylamine and beta-(2-thienyl) isopropylamine.' ACTA POLONIAE PHARMACEUTICA vol. 35, no. 2, 1978, pages 175 - 179, XP008181113
- BEBAN, MARC ET AL.: 'Preparation of an Imidazole-Conjugated Oligonucleotide.' BIOCONJUGATE CHEMISTRY vol. 11, no. 4, 2000, pages 599 - 603, XP055290934
- PODYMINOGIN, MIKHAIL A. ET AL.: 'Synthetic RNA-cleaving molecules mimicking ribonuclease A active center. Design and cleavage of tRNA transcripts.' NUCLEIC ACIDS RESEARCH vol. 21, no. 25, 1993, pages 5950 - 5956, XP055290935
- SCHUHMANN, ELFRIEDE ET AL.: 'Bis[platinum(II)] and Bis[palladium(II)] Complexes of alpha,omega-Dicarboxylic Acid Bis(1,2,4-triaminobutane-N4) Amides.' INORG. CHEM. vol. 34, no. 9, 1995, pages 2316 - 2322, XP055290936
- FERRY, GILLES ET AL.: 'A zinc chelator inhibiting gelatinases exerts potent in vitro anti-invasive effects.' EUROPEAN JOURNAL OF PHARMACOLOGY vol. 351, no. 2, 1998, pages 225 - 233, XP055290938
- GUPTA, ANIL ET AL.: 'Lipophilic complexones. Part 6. Synthesis of di- and tri-armed ligands containing pyridine and imidazole moieties.' J. CHEM. RESEARCH, SYNOPSES vol. 6, 1994, pages 206 - 207, XP008181114
- ROZHKOVA E. A. ET AL.: 'Vzaimosvyaz mezhdu konformatsiei i antioksidantnymi svoistvami v ryadu topokhimicheskikh analogov karnozina i kartsinina s razlichnymi N-atsilnymi zamestitelyami.' BIOORGANICHESKAYA KHIMIYA vol. 22, no. 10-11, 1996, pages 838 - 845, XP055290942
- None

## Description

The present invention relates to novel biologically active compounds, in particular, to dicarboxylic acid bisamide derivatives or pharmaceutically acceptable salts thereof, which are able to form complexes with, or chelate metal ions. The invention also relates to a use of said compounds as an agent for the prevention and/or treatment of cardiovascular, viral, oncological, neurodegenerative and inflammatory diseases, diabetes, age-related diseases, diseases caused by microbial toxins, alcoholism, alcoholic cirrhosis, anaemia, porphyria cutanea tarda, and transition metal salt poisoning.

## Background

Metal ions are very important both in functioning of cells and the whole organism, and in the development of pathologies.

There are known drugs, the activity of which is determined by their ability to chelate metal ions. In this context, non-toxic compounds are currently being researched which are able to chelate metal ions with a high efficiency and selectivity and which are suitable for biomedical applications.

Compounds with chelating ability have been found among the various classes of compounds, such as mono- and dithiols, disulfides, azo compounds, nitrosoaromatic compounds, polyaminocarboxylic acid derivatives, thiosemicarbazones, pyridoxal isonicotinoyl hydrazone, quinoline, adamantane, pyrogallol, phenanthroline, thiopyrophosphates, etc. In addition, they have been found among other natural compounds such as, for example, carnosine, phytin and pectin. Compounds having several functional groups, which are able to act as electron donors in complex formation reactions, are of greatest interest. Thus, such compounds can act as ligands which specifically interact with metal ions or groups of metals.

Complex forming agents widely known today are polyaminocarboxylic acid derivatives (e.g., EDTA), D-penicillamine and polycyclic cryptands, which are successfully used in heavy metal poisoning therapy. In some iron-excessive conditions and hemochromatosis, deferoxamine is used as an iron chelator. In addition, chelators may be useful in therapy of pathologies associated with high calcium level conditions, for example, arthroses, atherosclerosis and renal lithiasis. It is also known that chelation therapy prevents cholesterol accumulation and restores its level in the blood, lowers the blood pressure, allows avoiding angioplasty, suppresses undesirable side effects of certain cardiac drugs, removes calcium from cholesterol plaques, dissolves thrombi and restores the blood vessel elasticity, normalizes arrhythmia, prevents aging, restores cardiac muscle force and improves cardiac function, increases the intracellular potassium concentration, regulates the mineral metabolism, is useful in the treatment of Alzheimer's disease, prevents cancer, improves the memory, and has a plurality of other positive effects. However, strong chelators currently used in chelation therapy have, in general, a toxic effect which manifests itself mainly in damage of the mucous membrane of the small intestine and in kidney dysfunction. In some cases, rapid administration of large amounts of known chelators can impair muscular irritability and blood clotting. Furthermore, strong chelators can interact with valuable bioelements (Na, K, Ca, Mg, and Ca) and can change the activity of vitally important metalloenzymes [Zelenin K.N. "Chelators in medicine," Soros Educational Journal, 2001, v.7, No.1, pp.45-50].

In this context, novel high-effective chelators are currently being researched which posess chelating ability that is sufficient for achieving a biological effect *in vivo,* and are free of side effects.

The idea of chelation therapy of viral diseases, such as HIV, human papilloma, herpes, hepatitis and the like, is especially actual.

Zinc-binding sites (so-called "zinc fingers") in the structure of viral proteins are considered to be potential targets.

Today, several compounds have been found to affect the "zinc finger" structure of important proteins of these viruses.

The article of Andreas J.K., Bioorganic & Medicinal chemistry, 2003, v.11, pp.4599-4613, discloses an adamantane derivative with trivial name Bananin which is a zinc-ion chelator. The compound is assumed to be chemically suitable for removing zinc from the HIV protein NCp7. Azodicarbonamide being an azoderivative that is supposed to exhibit the above-mentioned mechanism of action, is currently in phase I/II clinical trials against progressive AIDS.

The article of Rice W.G., Schaeffer C.A., Harten B., Nature, 1993, v.4, pp.473-475, discloses 3-nitrozobenzamide which is able to remove zinc from the HIV protein NCp7 by inhibiting the replication of HIV and suppressing its pathogenicity *in vitro* and *in vivo.*

The "zinc finger"-like site of the human papilloma viral protein E6 was also selected as a target. This virus is a potential mediator in the etiology of cervical carcinoma.

The article of Beerheide W., Bernard H.-U., Tan Y.-J., Ganesan A.J., National Cancer Institute, 1999, v.91, No. 14, pp.1211-1220, describes *in vitro* studies of aza compounds, and disulfide and nitrozo aromatic derivatives. 4,4-Dithiodimorpholine type compounds have been shown to provoke the release of zinc ions. As a result, a change is observed in the structure of the viral protein and impairment of its functions associated with the biology and pathology of human papilloma virus. However, clinical trials of said compounds are not yet completed, and their effectiveness can

be judged only on the basis of *in vitro* studies.

The above-indicated compounds are assumed to be potential targets for the development of medicaments against cervical cancer, candyloma acuminate, and latent genital papillomavirus infections. Hepatitis C virus is one of the most common human pathogens. Modern therapy of hepatitis C is based almost solely on the use of interferon and its combination with the nucleoside analog ribavirin [Kozlov M.V., Polyakov K.M., Ivanov A.V., Biochemistry, 2006, v.71, No.9, pp.1253-12594]. It should be noted that the therapy is not highly effective.

As for the development of therapeutic agents against hepatitis C virus, one of targets is NS3-serine proteinase having a zinc site that is important for the maintenance of its structural stability [Andrea Urbani, Renzo Bazzo, Maria Chiara Nardi, Daniel Oscar Cicero, Raffaele De Francesco, J. Biol. Chem., 1998, v.273, No. 30, pp. 18760-18769]. Some literary sources report that the inhibition or alteration of its activity by using compounds with zinc removal capacity is a promising strategy of controlling a hepatitis C virus disease.

The article of Timothy L. Tellinghuisen, Matthew S. Paulson, Charles M. Rice, J. Virology, 2006, v. 80, No. 15, pp. 7450-7458, teaches that metal chelators (EDTA and 1,10-phenanthroline), which were assessed relative to NS2/3 auto-cleavage, are effective inhibitors of proteases. In addition, there is information that the effect of 1,10-phenanthroline was associated, in this case, exactly with chelation of zinc.

The article of Sperandio D., Gangloff A.R., Litvak J. Goldsmith R., Bioorg. Med. Chem. Lett., 2002, v.12, No. 21, pp.3129-3133, discloses screening of bisbenzimidazole compounds, aimed to seek for inhibitors of the hepatitis C virus NS3/NS4A serine protease, and mentions that said screening has also resulted in the identification of a corresponding powerful $Zn^{2+}$-dependent inhibitor.

Another attractive target in the development of novel approaches to the therapy of hepatitis C can be the hepatitis C virus RNA-dependent RNA polymerase (viral protein NS5B) comprising a zinc-binding site [Timothy L. Tellinghuisen, Matthew S. Paulson, Charles M. Rice, J. Virology, 2006, v. 80, No. 15, pp. 7450-7458].

Currently known inhibitors of the hepatitis C virus RNA-dependent RNA polymerase can be conventionally divided into two main classes: nucleoside derivatives and various non-nucleoside inhibitors [Maria Bretner, Acta biochemica polonica, 2005, v.52, No. 1, pp. 57-70]. In addition, it has been found that pyrogallol derivatives inhibit the activity of the indicated enzyme. It is remarkable that the inhibition mechanism for pyrogallol derivatives is assumed to be based on the chelation of magnesium cations which are involved in the catalytic action at the phosphoryl transfer step [Kozlov M.V., Polyakov K.M., Ivanov A.V., Biochemistry, 2006, v.71, No. 9, pp. 1253-12594].

Diseases caused by herpes viruses are widely spread. Thus, there are known several human herpes viruses, such as herpes simplex viruses 1 and 2 (HSV-1 and HSV-2), cytomegalovirus (CV), varicella zoster virus, and Epstein-Barr virus. Their destructive actions on the central nervous system cause diseases, such as encephalitis and meningitis. There is a large interest in the studies of an effect of zinc-chelating compounds, for example diethylenetriaminopentaacetic acid, on the inhibition of human cytomegalovirus replication *in vivo* [Kanekiyo M., Itoh N., Mano M., Antiviral Res., 2000, v. 47, pp. 207-214].

However, it is also remarkable that herpes viruses, similar to the above-mentioned viruses, comprise proteins with a "zinc finger"-like moiety. Chemical modifications of the "zinc finger" structure can lead to the release of zinc and functional changes in the viral proteins [Yan Chen, Christine M. Livingston, Stacy D. Carrington-Lawrence, J. of Virology, 2007, v.81, No. 16, pp. 8742-8751].

The "zinc finger" structure can serve as a target for new generation antiviral therapy. A number of such compounds are already found. However, today their efficiency can be judged only on the basis of *in vitro* studies.

The aforesaid information suggests the importance of metal ions both for the normal functioning of cells and the organism as a whole and for the development of pathologies. The capacity of some compounds to chelate metal ions defines research strategy to developmedications effective in the treatment of various diseases, including viral diseases.

The article of Megan Whitnall, Jonathan Howard, Prem Ponka, "A class of iron chelators with a wide spectrum of potent antitumor activity that overcomes resistance to chemotherapeutics", PNAS, 2006, v. 103, No. 40, pp. 14901-14906, discloses effective iron chelators which exhibit a high proliferative and anticancer activity similar to the activity of known cytostatics and which are potential for clinical trials.

Kik K., Szmigiero L., "Dexrazoxane (ICRF-187) - a cardioprotectant and modulator of some anticancer drugs", Postepy Hig Med Dosw Online, 2006, v. 60, pp. 584-590, reports that some iron chelators can be used as "helpers" in anticancer therapy, owing to their cardioprotective action.

The chelation of copper ions inhibits angiogenesis and slows down the tumor growth [Yu Yu, Jacky Wong, David B. Loveioy, "Chelatorsat the cancer coalface: Desferrioxamine to Triapine and Beyond", Clin. Cancer Res., 2006, v. 12, p. 6876-6883].

The zinc chelator, clioquinol, causes apoptosis of human cancer cells through the binding of $Zn^{2+}$ [Haijun Yu, Yunfeng Zhou, Stuart E. Lind, "Clioquinol targets zinc to lysosomes in human cancer cells", Biochem. J., 2009, v. 417, pp. 133-139].

Chelators are used as inhibitors of aldehyde dehydrogenase in the treatment of alcoholism [Shian S.G., Kao

Y.R., Wu F.Y., Wu C.W., "Inhibition of invasion and angiogenesis by zinc-chelating agent disulfiram", Mol. Pharmacol., 2003, v. 64(5), pp. 1076-84] and alcoholic cirrhosis, iron-excessive anemia, and porphyria cutanea tarda [Schroterova L., Kaiserova H., Baliharova V., "The effect of new lipophilic chelators on the activities of cytosolic reductases and P450 cytochromes involved in the metabolism of anthracycline as antibiotics: studies in vitro", Physiol. Res., 2004, v. 53(6), pp. 683-691].

The activity mechanism of some antioxidants is chelating transition metal ions (Fe, Cu), which sauses reduction in metal-dependent lipid peroxidation [Babizhayev M.A., Seguin Marie-C., Gueynej J., Evstigneev R.P., Ageyeva E.A., Zheltuchina G.A., "L-Carnosine(-alanyl-L-histidine) and carcinine f-alanylhistamine) act as natural antioxidants with hydroxyl-radical-scavenging and lipid-peroxidase activities", Biochem. J., 1994, v. 304, pp. 509-516] .

The use of antioxidants can lead to cataract resolution, elimination of retinal diseases, reduction in the need for insulin in diabetics, removal of skin pigmentation, and elimination of stroke sequelae symptoms. Chelation is also useful in the treatment of inflammatory diseases, such as osteoarthritis and rheumatoid arthritis [Zelenin K.N., "Chelators in medicine", Soros Educational Journal, 2001, v.7, No.1, pp.45-50].

Chelators can be used in medicine as chelating agents for transportation and easy extraction of arsenic, mercury, antimony, cobalt, zinc, chromium, and nickel from the organism [Zholnin A.V., "Complex compounds", Chelyabinsk: ChGMA, 2000, p. 28].

Botulinus toxin is known to be inhibited by chelating zinc ions [Anne C., Blommaert A., "Thio-derivede disulfides as potent inhibitors of botulinum neurotoxin B: implications of zinc interaction", Bioorg. Med. Chem., 2003, v. 11(21), pp. 4655-60]. In addition, chelation provides protection in gaseous gangrene therapy[Zelenin K.N., "Chelators in medicine", Soros Educational Journal, 2001, v.7, No.1, pp.45-50].

Chelation therapy is used in the treatment of neurodegenerative diseases, in particular Alzheimer's disease, to improve memory [Bossy-Wetzel E., Schwarzenbacher R., Lipton S.A., "Molecular pathways to neurodegeneration", Nat. Med, 2004, v. 10, pp. 2-9]; Parkinson's disease [Kevin J. Barnham, Colin L. Masters, Ashley I. Bush, "Neurodegenerative diseases and oxidative stress", Nature Reviews Drug Discovery, 2004, v. 3, pp. 205-214]; Wilson's disease [Yu Yu, Jacky Wong, David B. Lovejoy, "Chelators at the Cancer Coalface: Desferrioxamine to Triapine and Beyond", Clin. Cancer Res., 2006, v. 12, pp. 6876-6883]; Huntington's disease [Whitnall M., Richardson D.R., "Iron: a new target for pharmacological intervention in neurodegenerative diseases", Semin Pediatr Neurol, 2006, v. 13, pp. 186-197]; amyotrophic lateral sclerosis [Kevin J. Bernham, Colin L. Masters, Ashley I. Bush, "Neurodegenerative diseases and oxidative stress", Nature Reviews Drug Discovery, 2004, v. 4], and prion diseases [Daniel L. Cox, Jianping Pan, Rajiv R.P. Singh, "A Mechanism for Copper Inhibition of Infection Prion Conversion", Biophysical Journal, 2006, v. 91, L11-L13]. Chelators prevent the development of cancer [Megan Whitnall, "A class of iron chelators with a wide spectrum of potent antitumor activity that overcomes resistance to chemotherapeutics", PNAS, 2006, v. 103, No. 40, p. 14901-14906].

Among known chelators, derivatives of heterocyclic compounds (for example, imidazole) comprising imido and amido groups are of special importance.

The article of M.A. Podyminogin, V.V. Vlassov, "Synthesis RNA-cleaving molecules mimicking ribonuclease A active center. Design and cleavage of tRNA transcrints", Nucleic Acids Research, 1993, v. 21, No. 25, pp. 5950-5956, teaches a bis-histamine derivative of glutaric acid that can serve as a model of the active center of nucleases and exhibits a weak activity in the digestion of RNA molecules.

Elfriede Schuhmann et al. (see "Bis[platinum(II)] and Bis[Palladium (II)] complexes of α,ω-Dicarboxylic Acid Bis(1,2,4-triaminobutane-N4)-Amides", Inorg. Chem., 1995, v. 34, pp. 2316-2322) describe glutaric and adipic acid bis-histamine derivatives which are intermediates for the synthesis of complexes with platinum and palladium:

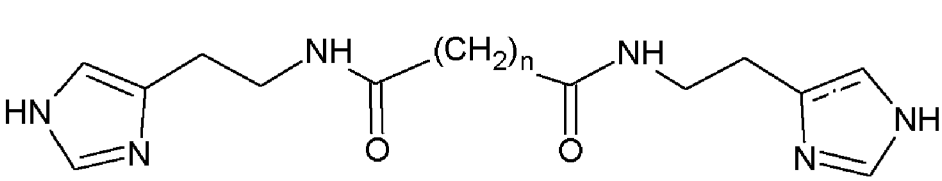

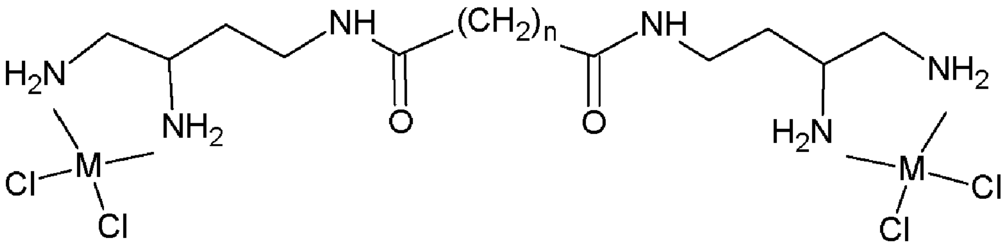

,

wherein n is 3-6 and 8; and
M is Pt and Pd.

The method of synthesis of $N^1$,$N^1$-glutaryl-bis(histamine), comprising contacting histamine dihydrocloride with glutaric acid dichloroanhydride in dimethylformamide in the presence of 4-fold excess of triethylamine is described in the article of Elfriede Schuhmann et al., "Bis[platinum(II)] and Bis[Palladium (II)] complexes of $\alpha$,$\omega$-Dicarboxylic Acid Bis(1,2,4-triaminobutane-N4)-Amides", Inorg. Chem., 1995, v. 34, pp. 2316-2322.

The inventors have found that a glutaric acid bis-histamine derivative, in particular $N^1$,$N^1$-glutaryl-bis(histamine), is capable of forming complexes with metal ions.

Thus, the objective of the present invention is to provide biocompatible heterocyclic metal ion chelators and the use thereof as a medicament for the treatment and/or prevention of various diseases by exploiting the capacity of the claimed compounds to chelate metal ions.

Another objective of the present invention is to provide simple methods for preparing such compounds by using available reactants.

## Summary of the invention

The present invention relates to dicarboxylic acid bisamide derivatives of general formula I:

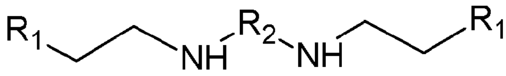
,

wherein

$R_1$ is a group selected from:

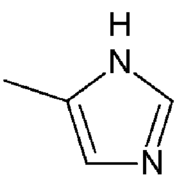
,
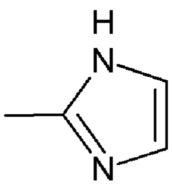
,
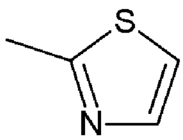
,
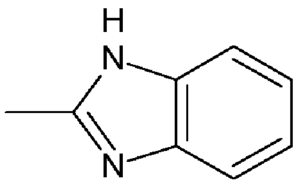
,

and

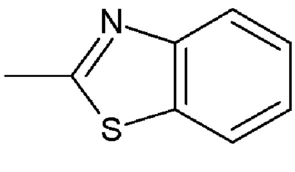
;

$R_2$ is a group selected from: $-C(O)-(CH_2)_0-(CO)-$, $-C(O)-(CH_2)_1-(CO)-$, $-C(O)-(CH_2)_2-(CO)-$, $-C(O)-(CH_2)_3-(CO)-$, $-C(O)-(CH_2)_4-(CO)-$, $-C(O)-CH_2-CH(CH_3)-CH_2-C(O)-$, $-C(O)-CH_2-C(CH_3)_2-CH_2-C(O)-$, or a group selected from:

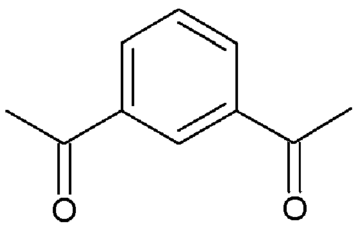
,
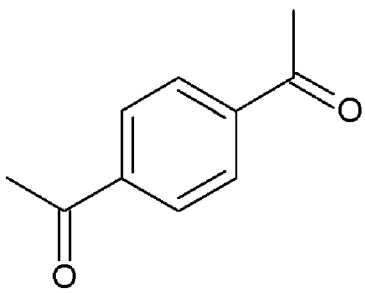
,

and

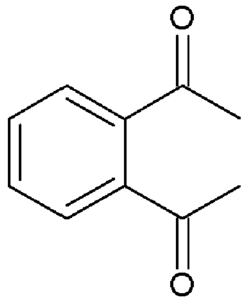

;

or a pharmaceutically acceptable salt thereof; with a proviso that the compound is not a compound, wherein:

$R_1$ is

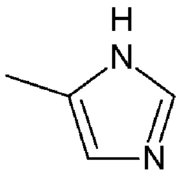

and $R_2$ is $-C(O)-(CH_2)_3-CO)-$; a compound, wherein:

$R_1$ is

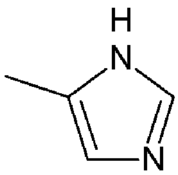

and $R_2$ is $-C(O)-(CH_2)_4-C(O)-$.

The present invention also relates to dicarboxylic acid bisamide derivatives of general formula I, which are able to chelate metal ions (Zn, Cu, Fe, Mg, Ca, etc.); and their use as an agent for the prevention and/or treatment of cardiovascular, viral, cancer, neurodegenerative disease, diabetes and its cardiovascular complications, diseases caused by microbial toxins, alcoholism and alcoholic cirrhosis, iron-excessive anaemia, porphyria cutanea tarda, and in the treatment of a disease associated with transition metal salt poisoning; wherein said composition comprises an effective amount of a compound of general formula I:

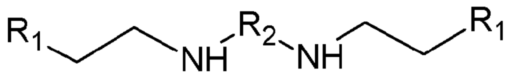

,

wherein

$R_1$ is a 5-membered unsaturated heterocyclic group comprising from 1 to 2 heteroatoms selected from N and/or S, optionally condensed with a 6-membered unsaturated cyclic group;
$R_2$ is $-C(O)-R_3-C(O)-$, wherein $R_3$ is $-(CH_2)_n-$ optionally substituted with one to two $C_1$-$C_6$alkyl groups or phenyl; and n is an integer from 0 to 4;
or a pharmaceutically acceptable salt thereof.

The present invention also relates to methods for preparing a compound of general formula I:

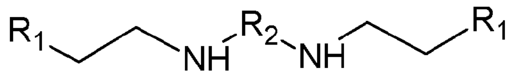
,

wherein

$R_1$ is a 5-membered unsaturated heterocyclic group comprising from 1 to 2 heteroatoms selected from N and/or S, optionally condensed with a 6-membered unsaturated cyclic group;
$R_2$ is -C(O)-$R_3$-C(O)-, wherein $R_3$ is $-(CH_2)_n-$ optionally substituted with one to two $C_1$-$C_6$alkyl groups or phenyl; and n is an integer from 0 to 4;
comprising steps of:

condensing a dicarboxilic acid of general formula II:

$$R_4O\text{-}C(O)\text{-}R_3\text{-}C(O)\text{-}OR_4,$$

wherein $R_3$ is $-(CH_2)_n-$ optionally substituted with one to two $C_1$-$C_6$alkyl groups or phenyl;
n is an integer from 0 to 4, and
$R_4$ is hydrogen or $C_1$-$C_6$alkyl;
with an amine of general formula III:

$$NH_2\text{-}(CH_2)_2\text{-}R_1,$$

wherein $R_1$ is a 5-membered unsaturated heterocyclic group comprising from 1 to 2 heteroatoms selected from N and/or S, optionally condensed with a 6-membered unsaturated cyclic group;
under heating, and optionally in the presence of a solvent, or in a molar ration of 1:2-2.5 in a tetrahydrofuran solution in the presence of a condensing agent.The claimed methods for preparing dicarboxylic acid heterocyclic bis-derivatives of general formula I are simple in implementation, conducted under quite mild conditions, are free of by-products, readily reproducible, and provide target products with a high yield (up to 82%) and of a high purity.

**Detailed description of the invention**

Preferred compounds according to the invention are compounds of general formula I:

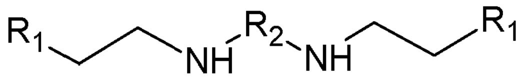

wherein $R_1$ is a group selected from:

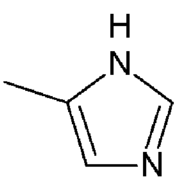
,
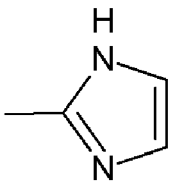
,
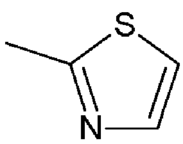
,
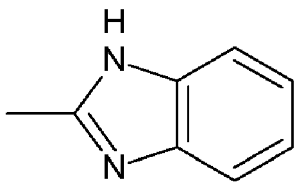
,

and

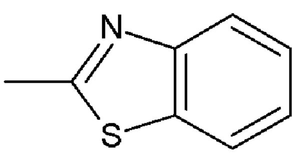
;

$R_2$ is a group selected from: $-C(O)-(CH_2)_0-(CO)-$, $-C(O)-(CH_2)_1-(CO)-$, $-C(O)-(CH_2)_2-(CO)-$, $-C(O)-(CH_2)_3-(CO)-$,

$-C(O)-(CH_2)_4-(CO)-$, $-C(O)(CH_3)-CH_2-C(O)-$, $-C(O)-CH_2-C(CH_3)_2-CH_2-C(O)-$, or a group selected from:

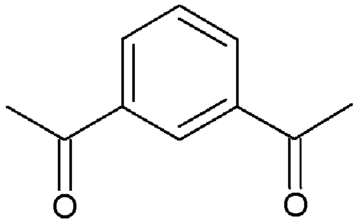 , 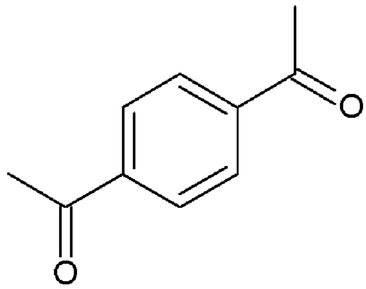 ,

and

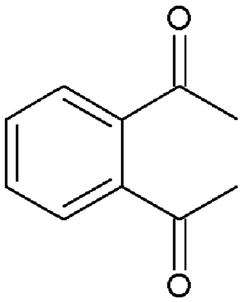 ;

with a proviso that the compound is not a compound, wherein:

$R_1$ is

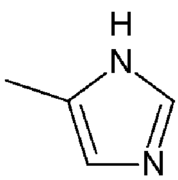

and $R_2$ is $-C(O)-(CH_2)_3-CO)-$; a compound, wherein:

$R_1$ is

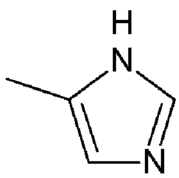

and $R_2$ is $-C(O)-(CH_2)_4-C(O)-$.

The most preferred compounds of the present invention are compounds given in Table 1.

Table 1

| Number of a compound | Structure |
|---|---|
| 1 | 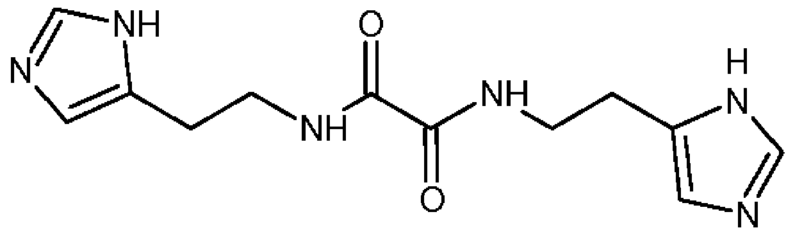 |
| 2 | 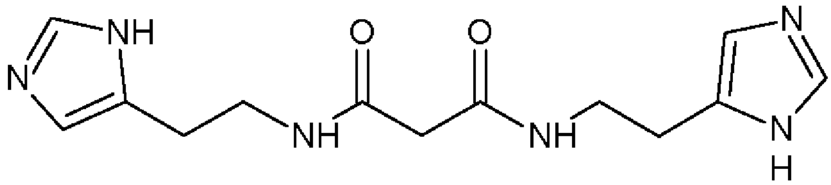 |
| 3 | 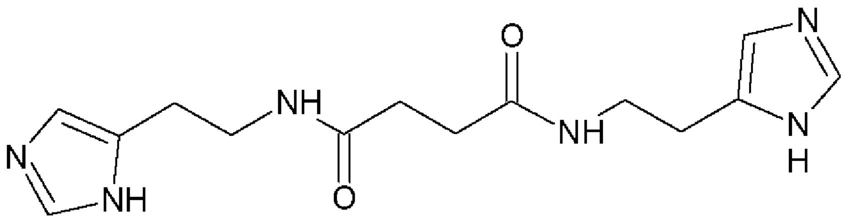 |
| 4 | 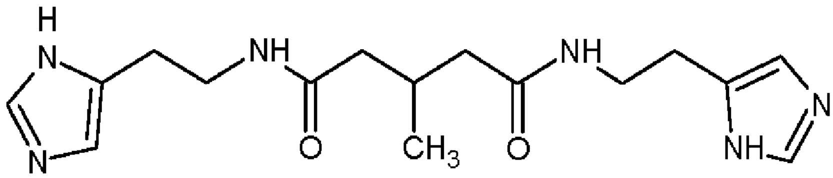 |
| 5 | 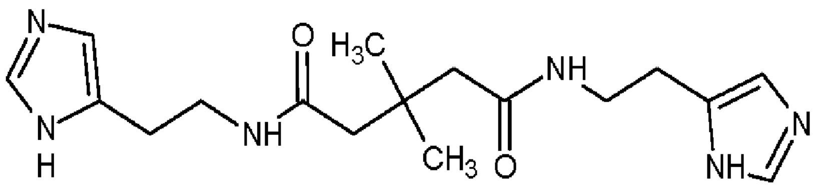 |
| 6 | 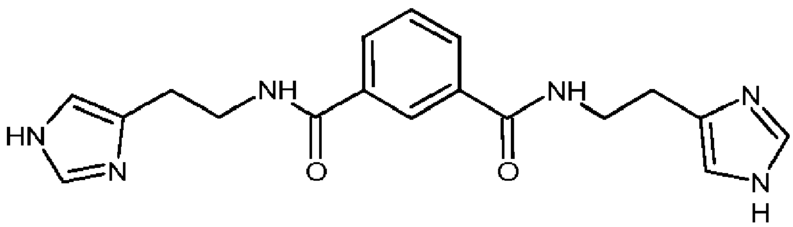 |
| 7 | 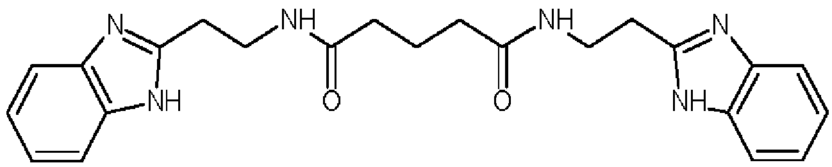 |
| 8 | 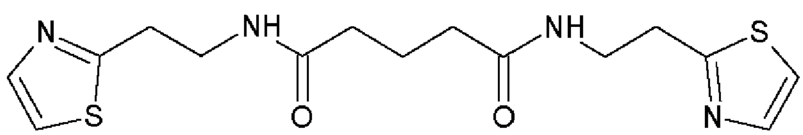 |

(continued)

| Number of a compound | Structure |
|---|---|
| 9 | 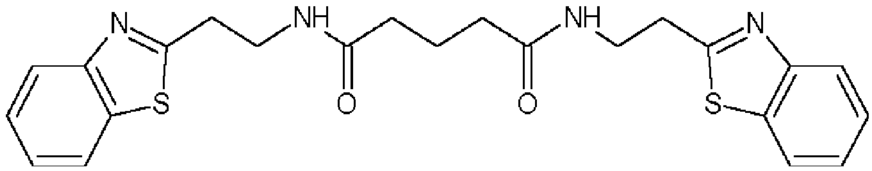 |
| 10 | 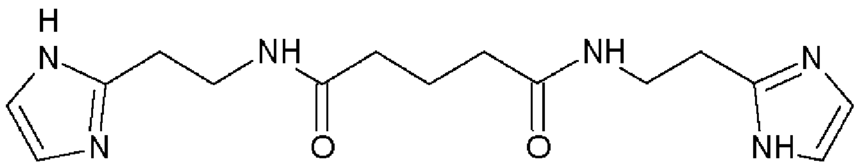 |

Pharmaceutically acceptable salts of the compounds according to the present invention may be selected from additive salts of organic acids (for example, formiate, acetate, maleate, tartrate, methanesulfonate, benzenesulfonate, toluenesulfonate, etc.), additive salts of inorganic acids (for example, hydrochloride, hydrobromide, sulphate, phosphate, etc.), and salts with amino acids (for example, an asparaginic acid salt, a glutamic acid salt, etc.), preferably chlorohydrates and acetates.

The most preferred known compounds that can be used in the pharmaceutical composition and method for the treatment according to the present invention are glutarimide derivatives represented in Table 2.

**Table 2**

| Number of a compound | Structure |
|---|---|
| 11 | 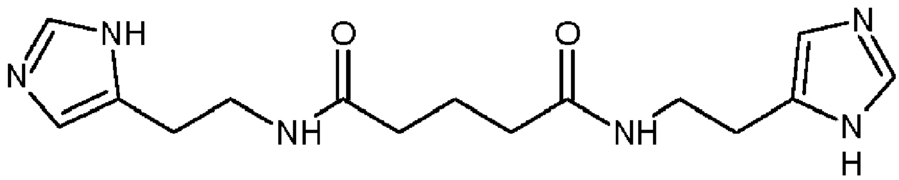 |
| 12 | 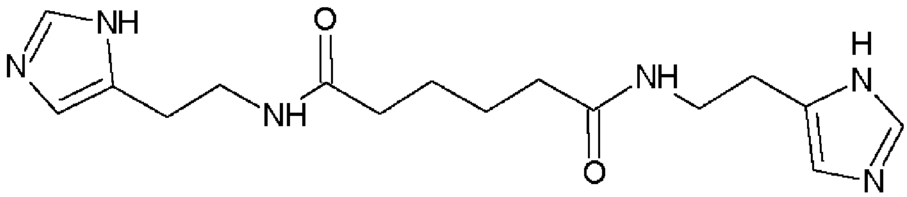 |

The compounds according to the present invention can be prepared by a method comprising condensing a dicarboxilic acid of general formula II:

$$R_4O\text{-}C(O)\text{-}R_3\text{-}C(O)\text{-}OR_4,$$

wherein $R_3$ is $-(CH_2)_n-$ optionally substituted with one to two $C_1$-$C_6$alkyl groups or phenyl;
n is an integer from 0 to 4, and
$R_4$ is hydrogen or $C_1$-$C_6$alkyl;
with an amine of general formula III:

$$NH_2\text{-}(CH_2)_2\text{-}R_1,$$

wherein $R_1$ is a 5-membered unsaturated heterocyclic group comprising from 1 to 2 heteroatoms selected from N and/or S, optionally condensed with a 6-membered unsaturated cyclic group;
under heating, optionally in the presence of a solvent.

It is preferable to use of dimethyl ether and to heat to 150-170°C; and it is more preferable to perform the condensation under boiling.

The solvent can be diglyme or an alcohol, more preferable isoamyl alcohol.

Another method for producing dicarboxylic acid bisamide derivatives of general formula I is a method comprising contacting a dicarboxylic acid of general formula II:

$R_4O-C(O)-R_3-C(O)-OR_4$,

wherein $R_3$ is $-(CH_2)_n-$ optionally substituted with one to two $C_1$-$C_6$alkyl groups or phenyl,
n is an integer from 0 to 4, and
$R_4$ is hydrogen,
with N-hydroxysuccinimide in the presence of N,N'-dicyclohexylcarbodiimide to form a corresponding bis-N-oxysuccinimide ester of general formula IV:

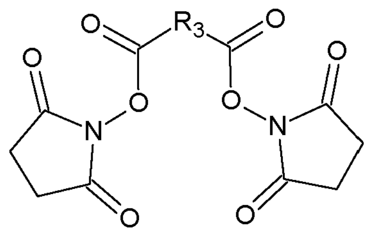

that is condensed with an amine of general formula III:

$NH_2-(CH_2)_2-R_1$,

wherein $R_1$ is a 5-membered unsaturated heterocyclic group comprising from 1 to 2 heteroatoms selected from N and/or S, optionally condensed with a 6-membered unsaturated cyclic group.

It is preferable to provide cooling to a temperature of 0-5°C.

Another method for producing dicarboxylic acid bisamide derivatives of general formula I is a method comprising contacting a dicarboxylic acid ester of general formula II:

$R_4O-C(O)-R_3-C(O)-OR_4$,

wherein $R_3$ is $-(CH_2)_n-$ optionally substituted with one to two $C_1$-$C_6$alkyl groups or phenyl,
n is an integer from 0 to 4, and
$R_4$ is $C_1$-$C_6$alkyl,
with hydrazine hydrate in an organic solvent to form bishydrazide of general formula V:

$H_2N-NH-C(O)-R_3-C(O)-NH-NH_2$,

treating the bishydrazide with sodium nitrite in an acidic medium at a temperature of about 0°C to form bisazide of general formula VI:

$N^--N^+=N-C(O)-R_3-C(O)-N=N^+-N^-$,

condensing the bisazide with an amine of general formula III:

$NH_2-(CH_2)_2-R_1$,

wherein $R_1$ is a 5-membered unsaturated heterocyclic group comprising from 1 to 2 heteroatoms selected from N and/or S, optionally condensed with a 6-membered unsaturated cyclic group, in an organic solvent.

An alcohol is preferable to be used as an organic solvent, and isopropanol is more preferable. The method is

simple, but is used when the number of methylene units in the initial dicarboxylic acid is more or equal to three since bisazides thereby obtained for corresponding acids with a lower number of methylene groups are not stable.

Dicarboxylic acid bisamide derivatives of general formula I also can be prepared by a method comprising condensing an imide of general formula VII:

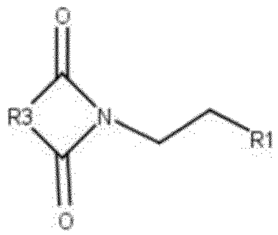

wherein $R_3$ is $-(CH_2)_n-$ optionally substituted with one or two $C_1$-$C_6$alkyl groups, and
n is an integer from 0 to 4,
with an equimolar amount of an amine of general formula III:

$$NH_2-(CH_2)_2-R_1,$$

wherein $R_1$ is a 5-membered unsaturated heterocyclic group comprising from 1 to 2 heteroatoms selected from N and/or S, optionally condensed with a 6-membered unsaturated cyclic group,
in an organic solvent under heating.

An alcohol is preferable to be used as an organic solvent, and isopropanol is more preferable. The condensation is preferable to be performed under boiling.

Another method of the present invention is a method for producing dicarboxylic acid bisamide derivatives of general formula I, comprising contacting a dicarboxylic acid of general formula II:

$$R_4O-C(O)-R_3-C(O)-OR_4,$$

wherein $R_3$ is $-(CH_2)_n-$ optionally substituted with one to two $C_1$-$C_6$alkyl groups or phenyl,
n is an integer from 0 to 4, and
$R_4$ is hydrogen,
with an amine of general formula III:

$$NH_2-(CH_2)_2-R_1,$$

wherein $R_1$ is a 5-membered unsaturated heterocyclic group comprising from 1 to 2 heteroatoms selected from N and/or S and optionally condensed with a 6-membered unsaturated cyclic group;
in a molar ratio of 1:2-2.5 in a tetrahydrofuran solution in the presence of a condensing agent, preferably carbonyldiimidazole.

The present invention also relates to a medicament and a pharmaceutical composition comprising a dicarboxylic acid bisamide derivative of general formula I, and to a method comprising administering a dicarboxylic acid bisamide derivative of general formula I to treat and/or prevent in a human and an animal viral diseases, such as diseases caused by hepatitis C virus, human papilloma virus, HIV or oncogenic RNA viruses, such as leukemia virus; cardiovascular diseases, including a disease caused by cytostatic cardiotoxicity, cholesterol accumulation, and hypertension; diseases associated with metal-dependent free-radical oxidation reactions, including age-related diseases, such as cataract, retinal disease, and skin pigmentation; stroke sequelae; atherosclerosis; inflammatory diseases, such as osteoarthritis, rheumatoid arthritis;

diabetes and its cardiovascular complications; neurodegenerative diseases, including Alzheimer's disease, Parkinson's disease, Wilson's disease, Huntington's disease, amyotrophic lateral sclerosis, and prion diseases;
oncologic diseases; diseases caused by microbial toxins, in particular botulism or gaseous gangrene;
alcoholism and alcoholic cirrhosis; iron-excessive anaemia, porphyria cutanea tarda; and transition metal salt poisoning.

The compounds according to the present invention are administered in an effective amount that provides a desired therapeutic effect.

The compounds of general formula I can be administered orally, topically, parenterally, intranasally, by inhalation, and rectally in a unit dosage form comprising non-toxic pharmaceutically acceptable carriers. The term "oral administration" used in the present invention means subcutaneous, intravenous, intramuscular or intrathoric injection or infusion.

The compounds according to the present invention can be administered to a patient at a dose of from 0.1 to 100 mg/kg of body weight once daily, preferably at a dose of from 0.25 to 25 mg/kg one or more times a day.

In addition it should be noted that a particular dose for a particular patient depends on many factors, including the activity of a certain compound, patient's age, body weight, gender, general health condition and diet, the time and route of administration of a pharmaceutical agent and the rate of its excretion from the body, a specific combination of drugs, and the severity of a disease in an individual to be treated.

The pharmaceutical compositions according to the present invention comprise a compound of general formula (I) in an amount effective to achieve a desired technical result, and can be administered in a unite dosage form (for example, in a solid, semisolid, or liquid form) comprising the compounds according to the present invention as an active agent in a mixture with a carrier or an excipient suitable for intramuscular, intravenous, peroral and sublingual administration, administration by inhalation, intranasal and intrarectal administration. The active ingredient can be in a composition together with conventional nontoxic pharmaceutically acceptable carriers suitable for the manufacture of solutions, tablets, pills, capsules, coated pills, emulsions, suspensions, ointments, gels, and any other dosage forms.

As an excipient, various compounds can be used, such as saccharides, for example, glucose, lactose, of sucrose; mannitol or sorbitol; cellulose derivatives; and/or calcium phosphates, for example, tricalcium phosphate or calcium hydrophosphate. As a binder, the following compounds can be used, such as a starch paste (for example, corn, wheat, rice, or potato starch), gelatin, tragacanth, methylcellulose, hydroxypropylmethylcellulose, sodium carboxymethylcellulose, and/or polyvinylpyrrolidone. Optionally used disintegrants are the above-mentioned starches and carboxymethylstarch, crosslinked polyvinylpyrrolidone, agar-agar, or alginic acid or a salt thereof, such as sodium alginate.

Additives that can be optionally used are flowability-control agents and lubricants, such as silicon dioxide, talc, stearic acid and salts thereof, such as magnesium stearate or calcium stearate, and/or propylene glycol.

The core of a coted pill is usually coated with a layer that is resistant to the action of gastric acid. For this purpose a concentrated solution of saccharides can be used, wherein said solutions can optionally comprise gum arabic, talc, polyvinylpyrrolidone, polyethylene glycol, and/or titanium dioxide, and suitable organic solvents or a mixture thereof.

Stabilizers, thickening agents, colorants, and fragrances also can be used as additives.

As an ointment base, there are usable hydrocarbon ointment bases, such as white Vaseline and yellow Vaseline (Vaselinum album and Vaselinum flavum, respectively), Vaseline oil (Oleum Vaselini), and white ointment and liquid ointment (Unguentum album and Unguentum flavum, respectively), wherein solid paraffin or wax can be used as an additive providing a firmer texture; absorptive ointment bases, such as hydrophilic Vaseline (Vaselinum hydrophylicum), lanoline (Lanolinum), and cold cream (Unguentum leniens); water-removable ointment bases, such as hydrophilic ointment (Unguentum hydrophylum); water-soluble ointment bases, such as polyethylene glycol ointment (Unguentum Glycolis Polyaethyleni); bentonite bases; and others.

A base for gels may be selected from methylcellulose, sodium caboxymethylcellulose, oxypropylcellulose, polyethylene glycol or polyethylene oxide, and carbopol.

A base for suppositories can be a water-insoluble base such as cocoa butter; a water-soluble or water-miscible base, such as gelatin-glycerol or polyethylene oxide; or a combined base, such as a soap-glycerol base.

In preparing a unit dosage form, the amount of an active agent used in combination with a carrier can vary depending on a recipient to be treated and on a particular route of administration of a therapeutic agent.

For example, when the compounds according to the present invention are used in the form of a solution for injection, the amount of the active agent in this solution is up to 5 wt.%. A diluent may be selected from a 0.9% sodium chloride solution, distilled water, a Novocain solution for injection, Ringer's solution, a glucose solution, and specific solubilizing adjuvants. When the compounds according to the present invention are administered in tablet or suppository form, their amount is up to 200 mg per unit dosage form.

Dosage forms according to the present invention are prepared by conventional procedures, such as blending, granulation, forming coating pills, dissolution, and lyophilization.

The detailed description of the compounds according to the present invention, their preparation, and studies of their activity are disclosed in the following examples that are intended for purposes of illustration only and are not intended to limit the scope of the invention.

Examples of synthesis of glutarimide derivatives of general formula I

*Means and methods*

Identity of obtained compounds were assessed by the thin-layer chromatography (TLC) method on plates "Kieselgel 60 F254" ("Merck", German) in a solvent system of pyridine-acetic acid-water (20:6:11) (System A); A:ethylacetate (3:1) (1), and chloroform-methanol (9:1) (2).

Paper electrophoresis (electrophoresis paper of Kondopozhskii TsBK, 120x320 mm) was performed in a buffer (pH 5.1) consisting of pyridine-acetic acid-water (12:10:1000) in a chamber (150x320x150 mm) with a gradient of 15 V/cm for 1.5 hours.

Chromatograms and electrophoregrams were treated with chloro-tetramethylbenzene reagent and Pauly's reagent.

The melting point was measured with a melting point apparatus (Plant of lab. equipment, Klin, Russia).

Fourier IR spectra were recorded on a "Magna 750" spectrometer with KBr tablets ("Nicolet" (US)).

$^1H$ NMR spectra were registered on Bruker AMX-400 (German) and DPX-400 (German) spectrometers.

High-resolution mass-spectra were obtained on a time-of-flight-assisted mass-spectrometer by the method of matrix laser-desorption ionization with 2,5-dihydroxybensoic acid used as a matrix, on an Ultraflex mass spectrometer ("Bruker", German).

Shimadzu Analytical HPLC SCL10Avp LC/MS system was used for the analysis of multicomponent mixtures on a mass spectrometer PE SCIEX API 165 (150) (Canada).

Analytical-scale reversed phase HPLC was performed on a Shimadzu HPLC chromatograph under the following conditions: Luna C18 (2) 100 A column, 250x4.6 mm (No. 599779-23), an elution gradient in a system of a phosphate buffer solution (pH 3.0):methanol (condition A); and on "System Gold" chromatographer (Beckman, US) with UV-detector ($\lambda$=220 nm), Phenomenex "Gemini" C-18 column, 150x2,0 mm, a mobile phase flow gradient in a 0.05 M phosphate buffer (pH 3.0), 90% MeCN in water, an elution rate of 0.25 ml/min, and 10 $\mu$l injection (condition B).

Example 1

Bis-1,5-($N^{\beta}$-histaminyl)glutaric acid; (N,N'-bis-[2-(1H-imidazol-4-yl)ethyl]pentanediamide (Compound 11).

Histamine (8 g; 0.072 mol) was added to glutaric acid dimethyl ester (5 g; 0.031 mol) and heated at 170°C for 3.5-4 hours until the evaporation of methyl alcohol was completed. The completeness of the reaction was checked by a TLC or electrophoresis method. Then the reaction mixture was suspended in isopropyl alcohol and allowed to stand for 24 hours at +4°C. The product was separated, washed with isopropyl alcohol, and dried. Yield was 6.8 g (69%). $R_f$ 0.42 (1). $E^+$49 mm. M.p. 166-168°C. LC/MS: an individual peak at a retention time of 0.3 min, $[M+H]^+$=319. HPLC under condition A: an individual peak at a retention time of 3.58 min. $^1H$ NMR (400.13 MHz, DMSO-$d_6$, $\delta$, m.d., J/Hz): 1.70 (quint, 2H, $CH_2C\underline{H_2}CH_2$, J=7.3 Hz), 2.03 (t, 4H, $C\underline{H_2}CH_2C\underline{H_2}$, J=7.3 Hz), 2.61 (t, 4H, $CC\underline{H_2}CH_2N$, J=7.5 Hz), 3.26 (quint, 4H, $CCH_2C\underline{H_2}N$, J=7.5 Hz), 6.76 (b.s, 2H, $CC\underline{H}$), 7.50 (s, 2H, $NC\underline{H}N$), 7.94 (b.t., 2H, $N\underline{H}$). Fourier-IR spectrum (in a KBr-table, $\nu$, $cm^{-1}$) : 1651 ($\nu$ C=O amide I), 1580 ($\delta$ NH amide II), 1425 ($-CH_2-CO-$) .

Found, %: C, 56.40; H, 6.86; N, 26.31. $C_{15}H_{22}N_6O_2$. Calculated, %: C, 56.59; H, 6.96; N, 26.40.

Example 2

Bis-1,4-($N^{\beta}$-histaminyl)succinic acid; (N,N'-bis-[2-(1H-imidazol-4-yl)ethyl]butanediamide) (compound 3)

Histamine (12.2 g; 0.11 mol) was added to succinic acid (5.5 g; 0.046 mol) and heated at 170°C for 3.5-4 hours until the evaporation of methyl alcohol was completed. The completeness of the reaction was checked by a TLC or electrophoresis method. Then the reaction mass was suspended in 50 ml of water and allowed to stand for 24 fours at +4°C. The product was separated, washed with water, and dried. Yield was 10.7 g (76%) . $R_f$ 0.51 (1). $E^+$51 mm. M.p. 230-231°C. $[M+H]^+$ 304.95. $^1H$-NMR ($D_2O$): $\delta$, m.d.: 2.48 (s, 4H, $CH_2$-Suc), 2.80-2.84 (t, 4H, $\beta$-$CH_2$-HA), 3.44-3.48 (t, 4H, $\alpha$-$CH_2$-HA), 6.97 (s, 2H, 5-CH-Im), 7.78 (s, 2H, 2-CH-Im). Fourier-IR spectrum (in a KBr-table, $\nu$, $cm^{-1}$) : 1634 ($\nu$ C=O amide I), 1583 ($\delta$ NH amide II), 1429 ($-CH_2-CO-$). Found, %: C, 55.40; H, 6.66; N, 27.31. $C_{14}H_{20}N_6O_2$. Calculated, %: C, 55.25; H, 6.62; N, 27.61.

Example 3

Bis-1,3-($N^{\beta}$-histaminyl)malonicacid;(N,N'-bis-[2-(1H-imidazol-4-yl)ethyl]propanediamide) (compound 2)

Histamine (9.0 g; 0.081 mol) was added to a malonic acid diethyl ester (6.4 g; 0.039 mol) and heated at 170°C

for 2.5-3 hours until the evaporation of methyl alcohol was completed. The completeness of the reaction was checked by a TLC or electrophoresis method. Then the reaction mass was suspended in 20 ml of water and allowed to stand for 72 fours at +4°C. The product was separated, washed with isopropyl alcohol, and dried. Yield was 5.0 g (44%) . $R_f$ 0.41 (1). $E^+$57 mm. M.p. 187-188°C. $^1$H-NMR ($D_2O$): δ, m.d.: 2.74-2.78 (t, 4H, β-$CH_2$-HA), 3.15 (s, 2H, $CH_2$-Mal), 3.41-3.44 (t, 4H, α-$CH_2$-HA), 6.78 (s, 2H, 5-CH-Im), 7.66 (s, 2H, 2-CH-Im). Fourier-IR spectrum (in a KBr-table, ν, $cm^{-1}$): 1643 (ν C=O amide I), 1574 (δ NH amide II), 1426 (-$\underline{CH_2}$-CO-). Found, %: C, 53.24; H, 6.51; N, 28.56. $C_{13}H_{18}N_6O_2$. Calculated, %: C, 53.78; H, 6.25; N, 28.95.

Example 4

Bis-1,2-($N^\beta$-histaminyl)oxalic acid; (N,N'-bis-[2-(1H-imidazol-4-yl)ethyl]ethanediamide) (compound 1)

Histamine (12.2 g; 0.11 mol) was added to a solution of an oxalic acid diethyl ester (7.3 g; 0.05 mol) in isoamyl alcohol (35 ml) and boiled for 4 hours. The completeness of the reaction was checked by a TLC or electrophoresis method. The reaction mixture was allowed to stand for 16 hours at +4C°. The residue was separated, washed with isopropyl alcohol, and dried. Yield was 10 g (72%) . $R_f$ 0.55 (1). $E^+$55 mm. M.p. 235-236°C. $^1$H-NMR (DMSO) : δ, m.d.: 2.66-2.72 (t, 4H, β-$CH_2$-HA), 3.33-3.41 (m, 4H, α-$CH_2$-HA), 6.81 (s, 2H, 5-CH-Im), 7.54 (s, 2H, 2-CH-Im), 8.78-8.84 (t, 2H, $\underline{NH}$-CO). Fourier-IR spectrum (in a KBr-table, ν, $cm^{-1}$): 1651 (ν C=O amide I), 1566 (δ NH amide II), 1425 (-$\underline{CH_2}$-CO-). Found, %: C, 52.24; H, 5.51; N, 29.97. $C_{12}H_{16}N_6O_2$. Calculated, %: C, 52.17; H, 5.84; N, 30.42.

Example 5

Bis-1,3-($N^\beta$-histaminyl)isophthalic acid (compound 6)

Isophthalic acid (5 g; 30 mmol) and N- hydroxysuccinimide (7.93; 69 mmol) were dissolved in dimethylformamide (30 ml). A cooled solution of N,N'-dicyclohexylcarbodiimide (14.24 g; 69 mmol) in dimethylformamide (10 ml) was added to the resulting solution cooled to 5°C, and the reaction mixture was allowed to stand for a night at 5°C. Urea was separated, washed with 15 ml of dimethylformamide, and dried. Based on the mass of the resulting urea, the yield of the reaction was considered to be equal to 80%. The solution of an isophthalic acid N-hydroxysuccinimide diester was cooled to 5°C, and then melted histamine (6.84 g; 61.6 mmol) was fed thereto batchwise. The reaction mixture was allowed to stand for 2 hours. The solvent was removed under vacuum. The yellow oil residue was dissolved in 120 ml of water and passed through a column with Amberlite IRA-96 (27x115). The fractions comprising the product were combined. Precipitated crystals of the target product were separated. The obtained crude product was recrystallized from a mixture of isopropanol (35 ml) and water (10 ml). The crystals of the product were filtrated, washed with water (3x30 ml) and isopropanol (2x13 ml), and dried. The target product was obtained in the form of white crystals. Yield was 1.55 g (18% based on histamine, or 14% based on isophthalic acid) . $R_f$ 0.42 (1) . M.p. 218-219°C. $E^+$40 mm. MS: $[M+1]^+$353. $^1$H-NMR: (300 MHz, DMSO-$d_6$) : δ, m.d.: 2.74-2.79 (t, 4H, β-$CH_2$-Ha), 3.46-3.52 (t, 4H, α-$CH_2$-Ha), 6.81 (s, 2H, 5-CH-Im), 7.52-7.56 (m, 3H, $ArH^{+2}$-CH-Im), 7.92-7.95 (d, 2H, ArH), 8.28 (s, 1H, ArH), 8.65 (b.s., 2H, -C(O)-NH-), 11.80 (b.s., 2H, -NH-). HPLC under condition B: an individual peak at a retention time of 12.5 min.

Example 6

Bis-1,5-($N^\beta$-histaminyl)glutaric acid; (N,N'-bis-[2-(1H-imidazol-4-yl)ethyl]pentanediamide) (compound 11)

$PCl_3$ (8 ml) was added batchwise to a solution of glutaric acid (13.2 g; 0.10 mol) in methanol (50 ml) under cooling and stirring. The solvent was removed from the reaction mixture under vacuum. The resulting residue was distilled off under vacuum. The amount of the resulting glutaric acid dimethyl ester with a boiling point of 110-112°C was 14.7 (92%).

Hydrazine hydrate (7.5 ml) was added to isopropyl alcohol (20 ml), heated to a boiling point and then the glutaric acid dimethyl ester (8 g; 0.05 mol) was added thereto dropwise, and the reaction mixture was allowed to stand for 16 hours at +20°C. The precipitated product was separated, washed with isopropyl alcohol, and dried. The amount of glutaric acid dihydrazide with a melting point of 210-212°C was 7.3 (91%).

Sodium nitrate (1.45 g; 0.021 mol) was added batchwise under stirring to a solution of the glutaric acid dihydrazide (1.61 g; 0.010 mol) in a mixture of ice (20 g) with hydrochloric acid (2.5 ml) and previously cooled chloroform (10 ml). The chloroform layer was separated, washed with water (10 ml), cooled to +4°C, and then isopropanol (5 ml) was added to a cooled histamine solution (2.6 g; 0.023 mol). The reaction mixture was allowed to stand for 2 hours at +20°C, and then the solvent was removed under vacuum. The resulting residue was dissolved in water (20 ml) and passed through a column with 50 ml of ion-exchange resin KU-2-20 ($H^+$-form). The resin was washed with a 0.5% ammonia solution,

collecting histamine-free fractions of the product. Eluates were combined, the solvent was removed under vacuum, and the resulting residue was recrystallized from isopropanol (2 ml) and dried. The yield was 1.8 g (55%) . $R_f$ 0.42 (1). $E^+$49 mm. M.p. 166-168°C. $^1$H-NMR ($D_2O$) : δ, m.d.: 1.73-1.78 (m, 2H, β-$CH_2$-Glt), 2.10-2.15 (t, 4H, α-$CH_2$-Glt), 2.78-2.82 (t, 4H, β-$CH_2$-HA), 3.43-3.48 (t, 4H, α-$CH_2$-HA), 6.94 (s, 2H, 5-CH-Im), 7.69 (s, 2H, 2-CH-Im). Fourier-IR spectrum (in a KBr table, ν, $cm^{-1}$) : 1651 (ν C=O amide I), 1580 (δ NH amide II), 1425 (-$CH_2$-CO-). Found, %: C, 56.40; H, 6.86; N, 26.31. $C_{15}H_{22}N_6O_2$. Calculated, %: C, 56.59; H, 6.96; N, 26.40.

Example 7

Bis-1,5-($N^β$-histaminyl)adipic acid; (N,N'-bis-[2-(1H-imidazol-4-yl)ethyl]hexanediamide) (compound 12)

Phosphorus trichloride (4 ml) was added dropwise under cooling to a solution of adipic acid (7.40; 0.05 mol) in methanol (25 ml). The solvent was removed from the reaction mixture under vacuum, and the resulting residue was distilled off under vacuum. The amount of the resulting adipic acid dimethyl ester with a boiling point of 115-117°C was 7.5 (86%).

The adipic acid dimethyl ester (7.5 g; 0.04 mol) was added dropwise to a solution of isopropyl alcohol (20 ml) and hydrazine hydrate (5 ml), heated to a boiling point, then the reaction mixture was allowed to stand for 16 hours at +20°C. The precipitate was separated, washed with isopropyl alcohol, and dried. The amount of the resulting adipic acid dihydrazide with a melting point of 182-182.5°C was 6.2 (83%).

Sodium nitrate (1.8 g; 0.026 mol) was added portionwise under stirring to a solution of the adipic acid dihydrazide (2.2 g; 0.013 mol) in a mixture of ice (30 g) with hydrochloric acid (3 ml) and cooled chloroform (15 ml). The chloroform layer was separated, washed with water (10 ml), and cooled to +4°C, then isopropanol (8 ml) was added to the cooled histamine solution (3 g; 0.027 mol). The completeness of the reaction was checked by a TLC or electrophoresis method. The reaction mixture was allowed to stand for 24 hours at +4°C, and the residue was separated, washed with isopropyl alcohol, recrystallized from water, and dried. The yield was 2.8 g (65%). $R_f$ 0.48 (1). $E^+$45 mm. M.p. 184-186°C. $[M+H]^+$ 332.92. $^1$H-NMR ($D_2O$): δ, m.d.: 1.37-1.41 (m, 4H, β-$CH_2$-Adip), 2.11-2.15 (m, 4H, α-$CH_2$-Adip), 2.74-2.78 (t, 4H, β-$CH_2$-HA), 3.40-3.44 (t, 4H, α-$CH_2$-HA), 6.88 (s, 2H, 5-CH-Im), 7.63 (s, 2H, 2-CH-Im). Fourier-IR spectrum (in a KBr table, ν, $cm^{-1}$) : 1646 (ν C=O amide I), 1581 (δ NH amide II), 1425 (-$CH_2$-CO-). Found, %: C, 57.45; H, 7.15; N, 24.97. $C_{16}H_{24}N_6O_2$. Calculated, %: C, 57.81; H, 7.28; N, 25.28.

The following compounds were prepared by the above-disclosed method:

| Number of a compound | Structural formula | Physical and chemical data |
|---|---|---|
| Compound 8 | 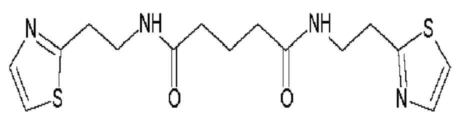 | MS: $[M+1]^+$=353. HPLC: under condition B, an individual peak at a retention time of 16.1 min. $^1$H-NMR (400 MHz, DMSO-$d_6$): δ, m.d.: 1.63-1.75 (2H, m, -$CH_2$), 1.96-2.11 (4H, t, J=7.46, 2$CH_2$), 3.06-3.16 (4H, t, J=6.97, $CH_2$), 3.35-3.46 (4H, dd, J1=6.61, J2=6.11, $CH_2$), 7.66-7.74 (2H, d, J=3.18, Ar), 7.66-7.74 (2H, d, J=3.18, Ar), 7.9-8.0 (2H, m, NH). |
| Compound 10 | 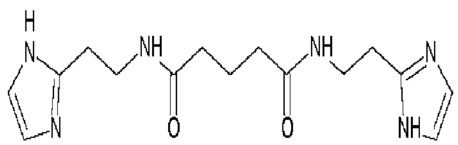 | LC/MS: an individual peak at a retention time of 0.3 min. $[M+H]^+$=319. HPLC under condition A: an individual peak at a retention time of 4.12 min. $^1$H NMR (400.13 MHz, DMSO-$d_6$, δ, m.d., J/Hz): 1.69 (quint, 2H, $CH_2CH_2CH_2$, J=7.3 Hz), 2.01 (t, 4H, $CH_2CH_2CH_2$, J=7.3 Hz), 2.73 (t, 4H, $CCH_2CH_2N$, J=7.5 Hz), 3.31 (quint, 4H, $CCH_2CH_2N$, J=7.5 Hz), 6.87 (s, 4H, CHN), 8.00 (b.t., 2H, NH). |

Example 8

Bis-1,4-($N^β$-histaminyl)succinic acid; (N,N'-bis-[2-(1H-imidazol-4-yl)ethyl]butanediamide) (compound 3)

Succinimide histamine (0.15; 0.78 mmol) was added to a solution of histamine (0.10 g; 0.9 mmol) in isopropyl alcohol (3 ml) and heated under boiling for 2.5-3 hours. The completeness of the reaction was checked by a TLC or electrophoresis method. Then the reaction mixture was cooled to room temperature and allowed to stand for 26 fours

at +4°C. The product was separated, washed with isopropyl alcohol, and dried. Yield was 0.20 g (81%). $R_f$ 0.44 (1). $E^+$51 mm. M.p. 231°C. $[M]^+$ 304.12. $^1$H-NMR ($D_2O$): $\delta$, m.d.: 2.48 (s, 4H, $CH_2$-Suc), 2.80-2.84 (t, 4H, $\beta$-$CH_2$-HA), 3.44-3.48 (t, 4H, $\alpha$-$CH_2$-HA), 6.97 (s, 2H, 5-CH-Im), 7.78 (s, 2H, 2-CH-Im). Fourier-IR spectrum (in a KBr table, $\nu$, $cm^{-1}$): 1634 ($\nu$ C=O amide I), 1583 ($\delta$ NH amide II), 1429 (-$CH_2$-CO-). Found, %: C, 6.57; N, 27.18. $C_{14}H_{20}N_6O_2$. Calculated, %: C, 55.25; H, 6.62; N, 27.61.

Example 9

Bis-1,5-(N-ethylamino-2-benzimidazolyl)glutaric acid; (N,N'- bis-[2-(1-Э-benzimidazol-2-yl)ethyl]pentanediamide) (compound 7)

Carbonyldiimidazole (23.4 g; 0.145 mol) was added to a solution of glutaric acid (8 g; 0.06 mol) in anhydrous tetrahydrofuran (500 ml) and stirred for 2 hours. Then benzimidazolyl ethylamine (21.6 g; 0.133 mol) was added to the reaction mixture. The resulting solution was stirred for 3 hours and allowed to stand at room temperature. The residue of the product was separated, washed with water, and dried. The yield was 21 g (82.9%). $R_f$ 0.21 (2). M.p. 170-170.5°C. $[M]^+$419. $^1$H-NMR (DMSO) : $\delta$, m.d.: 1.61-1.76 (m, 2H, $CH_2$), 1.95-2.07 (t, 2H, $CH_2$), 2.9-3.0 (t, 4H, $2CH_2$), 3.45-3.55 (quint, 4H, $2CH_2$), 7.06-7.14 (m, 4H, Ar), 7.39-7.54 (m, 4H, Ar), 7.98-8.08 (t, 2H, Ar), 11.6-12.7 (s, 2H, NH). HPLC under condition B: an individual peak at a retention time of 26.6 min.

The following compounds were prepared by the above-disclosed method:

| Number of a compound | Structural formula | Physical and chemical data |
|---|---|---|
| Compound 9 | 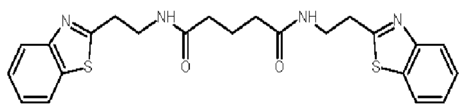 | MS: $[M+1]^+$=453. $^1$H-NMR (400 MHz, DMSO-$d_6$): $\delta$, m.d.: 1.65-1.77 (2H, m, $CH_2$), 2.0-2.1 (2H, t, J=7.46, $CH_2$), 3.16-3.26 (4H, t, J=6.97, $2CH_2$), 3.45-3.56 (4H, quint, J1=6.48, J2=5.87, $2CH_2$), 7.35-7.44 (1H, m, Ar), 7.44-7.52 (1H, m, Ar), 7.9-7.96 (2H, d, Ar, J=7.82), 7.96-8.02 (2H, t, NH, J=5.5). 8.02-8.07 (2H, d, Ar, J=7.82). HPLC under condition B: an individual peak at a retention time of 18.7 min |

*Study of the chelating ability of the claimed compounds*

The following studies have revealed that the claimed compounds of general formula I are able to form complexes with or chelate metal ions. They comprise several functional groups that can serve as donors in a complex formation process, for example, a carboxyl group, imido and amido groups, and are ligands specifically binding metal ions, for example, ions of zinc, copper, iron, calcium, and magnesium.

An important property of a ligand to form a complex is dissociation constants ($pk_n$) of studied compounds in an aqueous solution. The $pk_n$ values are determined by a potentiometric titration method. The calculation of dissociation constants are performed by the Schwarzenbach method [Grinberg A.A., "An introduction to the chemistry of complex compounds", fourth ed., Amend., L., Khimiya, 1971].

*pH-Potentiometric titration methods*

Titration was performed on an apparatus by using a laboratory ionomer I 120.1. A designed galvanic cell consisted of two electrodes, in particular an indicator glass electrode and a silver chloride comparison electrode. The pH value was measured with an accuracy of $\pm$0.2.

Initial 0.05 M solutions of $M^{z+}$ metal ions were prepared from nitrates of zinc ($Zn(NO_3)_2 \cdot 6H_2O$), calcium ($Ca(NO_3)_2 \cdot 4H_2O$), copper sulfate ($CuSO_4 \cdot 5H_2O$), magnesium chloride ($MgCl_2 \cdot 6H_2O$) and Mohr's salt ($(NH_4)_2Fe(SO_4)_2 \cdot 6H_2O$). Standardization of the initial solutions of $M^{z+}$ salts was performed by a complexometric titration method with trilon B ($M^{z+}=Zn^{2+}$, $Cu^{2+}$, $Mg^{2+}$, $Ca^{2+}$) and a spectrophotometric method at 490 nm by using o-phenanthroline ($M^{z+}=Fe^{2+}$).

A traditional method for determining acid dissociation step constants ($k_1$,..., $k_n$) by using pH-metric titration is the Schwarzenbach method.

A sample of a studied compound ($3.10^{-4}$ mol) was dissolved in 15.0 ml of a 0.5M aqueous $KNO_3$ solution. The

obtained 0.2M solution was titrated with a 0.05M aqueous solution of NaOH or HCl under stirring. The amount (V, ml) of the used titrant and the pH value of the solution were registered in each titration point. A titration step was equal to 0.5 ml, and 0.2 ml in the proximity to the equivalency point. Titration was continued until the pH value of the solution reached 11.0 to 11.5. The pH value of the solution corresponding to a=0.5 and/or a=1.5 was determined by the titration curve pH=f(a), and the amount of added gram-equivalents of the titrant was calculated in terms of 1 mole of a titrated compound.

The results of the determination of acid dissociation constants [$pk_n$] of the studied compounds by the Schwarzenbach method are given in Table 3.

Table 3

| pk Values of the studied compounds in an aqueous solution, calculated by the Schwarzenbach method (C = 0.02M; ionic strength of a solution = 0.5 ($KNO_3$); 22°C) | | |
|---|---|---|
| Number of a compound | $pk_1$ | $pk_2$ |
| 2 | 7.11(1) | 11.27(2) |
| 3 | 6.88 | 11.27(2) |
| 11 | 7.16(1) | 11.89(2) |
| 12 | 7.13(1) | 11.95(2) |
| (1) - titration with an 0.05M HCl aqueous solution.<br>(2) - titration with an 0.05M aqueous NaOH solution. | | |

Given the parameters, such as the amount of a studied compound required for potentiometric titration, a testing procedure and a constant calculation method, the Bjerrum method is most suitable and informative [Yu.P. Galaktionov, M.A. Chistyakov, V.G. Sevastiyanov, etc., "Step Stability Constants of Group IIIB Metal and Lanthanide Complexes with Acetylacetone in Aqueous Solution and the Solubility Products of Metal Triacetylacctonates", Journal of physical chemistry, 2004, v. 78, No. 9, pp. 1596-1604].

The method is based on the view that the process of complex formation is stepwise. A main assumption of the Bjerrum method is an assumption about the formation of only multinuclear complexes. The process of complex formation can be schematically described as follows:

$$M+L \leftrightarrow ML \quad K_1=[ML]/[M][L]$$

$$ML_{n-1}+L \leftrightarrow ML_n \quad K_n=[ML_n]/[ML_{n-1}][L]$$

According to the Bjerrum method, a studied compound is titrated with a NaOH solution twice: in the absence of and in the presence of a metal ion. The titration is performed under a high constant concentration of an indifferent electrolyte, which provides the solution with a constant ionic strength.

For these purposes, highly soluble salts are used, for example, sodium and potassium salts. Concentration step stability constants ($K_n$) are determined from experiments. In addition, the method provides information concerning the composition of a formed complex, in particular, the maximum possible number of bound ligands (n).

Traditional procedure for determining step stability constants for complexes by the Bjerrum method of pH-metric titration was performed as follows.

A sample of a studies compound ($3 \cdot 10^{-4}$ mol) was dissolved in 14.0 ml of a 0.5M aqueous $KNO_3$ solution. One ml of a 0.05M aqueous solution of an $M^{z+}$ salt was added under vigorous stirring. The systems were studied with a $M^{z+}/L$ ratio of 1:6. The pH value of a solution was fixed for coexisting ligands and metal ions. The mixture was titrated with a 0.05M NaOH aqueous solution within a broad range of pH values (up to pH 11.0-11.5). The amount of the used titrant ($V_{NaOH}$, ml) and the pH value of the solution were registered in each titration point. At the moment when a residue formed, the solution became turbid, or the color of the solution changed, the pH value, the nature of the change and the amount of NaOH spent for the titration were registered. The step of titration was 0.25 ml. The value of $lgK_n$ was determined by the Bjerrum curve $\bar{n}$=f(pA) for each semi-integer value of the function of complex formation $\bar{n}$=n-0.5.

Today the Bjerrum method is frequently used to study complex formation processes. This method provides for the determination of two parameters: the concentration of a free ligand under equilibrium conditions ($[L^-]$) and the function

of complex formation $\bar{n}$ which is the average number of ligands involved in the complex. Thus, using experimental data, the value $[L^-]$ can be calculated for each time point of the titration (in particular, $-lg[L^-]=pA$), and $\bar{n}$ can be calculated by equation (1). When a ligand has several dissociating groups, the $K_L$ value in equation (1) matches the value of the dissociation constant with the largest pk value.

The $K_n$ value is found from the Bjerrum curve (curve of the complex formation) $\bar{n}=f(pA)$ plotted according to equation (2) for each semi-integer value ($\bar{n}=n-0.5$) of the function of the complex formation. Thus, the step stability constant will be equal to the reciprocal concentration of a free ligand at a point of $\bar{n}=n-0.5$. Accordingly, the larger the $K_n$ value, the more stable a complex compound (chelate) is.

$$\bar{n} = \frac{c_{L^-} - [L^-]}{c_{M^{z+}}} = \frac{(10^{-pH} + K_L) \cdot C^0_{NaOH} \cdot V^{added}_{NaOH} - K_L \cdot n_L}{10^{-pH} \cdot n_{M^{z+}}} \qquad (1)$$

$$K_{st}n = \frac{1}{[L^-]} \qquad (2)$$

wherein $c_{L-}$ and $c_{Mz+}$ are the total concentrations of a ligand and a metal existing in a solution, mol/g;

$[L^-]$ is the concentration of free ligand ions, mol/g;
$K_L$ is the acid dissociation constant of a ligand;
$V^{added}_{NaOH}$ is the volume of a NaOH solution ( $C^0_{NaOH}$ , mol/l) at any time point of titration, added to the titrated mixture of $M^{z+}$ and L, l;
$n_L$ and $n_{Mz+}$ are the amounts of a ligand compound and a $M^{z+}$ salt in the titrated solution, mol.

The $lgK_1$ values found by the Bjerrum method for the complexes of the studied compounds with divalent metals are given in Tables 4 and 5. The $M^{z+}/L$ ratio of 1:6 is based on the data concerning the maximum possible coordination number for Zn(II) and Fe(II) (equal to six) and for Cu(II) (equal to four) [Claudia A. Blindauer, M. Tahir Razi, Simon Parsons, Peter J. Sadler, Polyhedron, 2006, v. 25, pp. 513-520].

Table 4

| The first stability constant of complexes with 1:1 compositions of studied compounds with divalent metal ions ($lgK_1$), found by the Bjerrum method (aqueous solution, $C_L$ = 0.02M; $M^{z+}/L$ = 1:6; ion strength = 0.5 ($KNO_3$), 22°C). | | | | | | |
|---|---|---|---|---|---|---|
| Number of a compound | The first stability constant of complexes with 1:1 compositions of studied compounds with divalent metal ions - $lgk_1$ | | | | | |
| | Zn(II) | Cu(II) | Fe(II) | Fe(III) | Mg(II) | Ca(II) |
| 2 | 4.84 | 4.17 | 5.08 | 5.29 | 2.59 | 2.06 |
| 3 | 5.40 | 5.32 | 5.95 | 6.59 | 4.36 | 4.57 |
| 11 | 5.05 | 4.39 | 6.27 | 6.60 | 2.82 | $\bar{n}$<0.5 |
| 12 | 5.46 | 4.62 | 6.40 | 6.10 | 3.72 | 3.60 |

Table 5 The step stability constant of complexes with 1:1 ratio of studied compounds with zinc ions, found by the Bjerrum method (aqueous solution, $C_L$ = 0.02 M; $M^{z+}/L$ = 1:6; ion strength = 0.5 ($KNO_3$), 22°C).

| Number of a compound | Step stability constant of complexes with 1:1 ratio of studied compounds with zinc ions | | |
|---|---|---|---|
| | The first stability constant, $lgk_1$ | The second stability constant, $lgK_2$ | The third stability constant, $lgK_3$ |
| 1 | 4.52 | 4.21 | 3.75 |
| 2 | 4.84 | 4.50 | 2.60 |

(continued)

| Number of a compound | Step stability constant of complexes with 1:1 ratio of studied compounds with zinc ions | | |
|---|---|---|---|
| | The first stability constant, $lgk_1$ | The second stability constant, $lgK_2$ | The third stability constant, $lgK_3$ |
| 3 | 5.40 | 5.05 | 3.80 |
| 6 | 5.62 | 5.15 | 4.04 |
| 8 | 6.25 | 5.80 | 5.05 |
| 10 | 5.50 | 5.24 | 4.48 |
| 11 | 5.05 | 4.70 | 2.87 |
| 12 | 5.46 | 5.03 | 3.38 |

Thus, the studies have shown that the studied compounds, in particular dicarboxylic acid bisamide derivatives, are effective complex formers, most of which have been found to have high values of $lgK_1 \geq 5$ for transition metal ions. In addition, the claimed compounds of general formula I have been shown to exhibit a slightly increased ability to form complexes with iron ions.

## Claims

**1.** A dicarboxylic acid bisamide derivative of general formula:

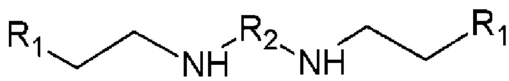
,

wherein:

$R_1$ is a group selected from:

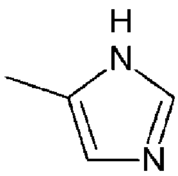
,
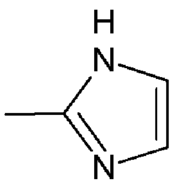
,
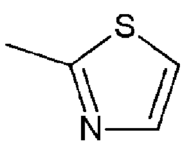
,
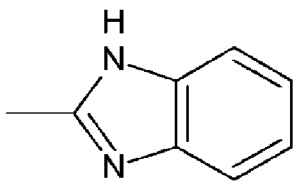
,

and

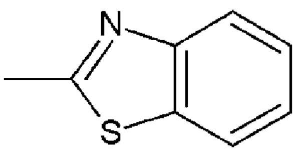
;

and

$R_2$ is a group selected from: $-C(O)-(CH_2)_0-C(O)-$, $-C(O)-(CH_2)_1-C(O)-$, $-C(O)-(CH_2)_2-C(O)-$, $-C(O)-(CH_2)_3-C(O)-$, $-C(O)-(CH_2)_4-C(O)-$, $-C(O)(CH_3)-CH_2-C(O)-$, and $-C(O)-CH_2-C(CH_3)_2-CH_2-C(O)-$, or a group selected from

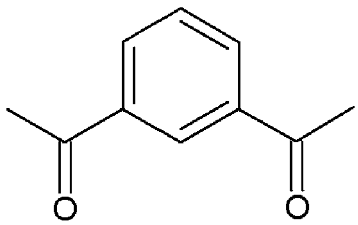, 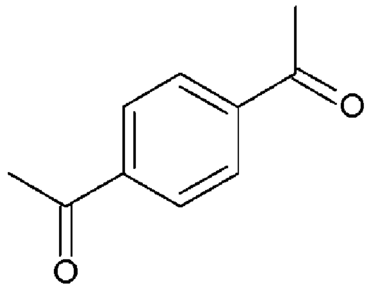, and 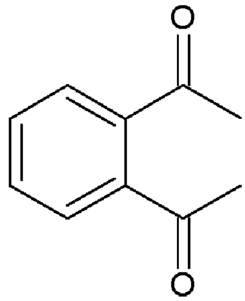

or a pharmaceutically acceptable salt thereof,
with a proviso that the compound is not
a compound, wherein:

$R_1$ is

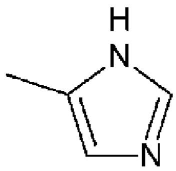

and $R_2$ is -C(O)-$(CH_2)_3$-C(O)-;
a compound, wherein:
$R_1$ is

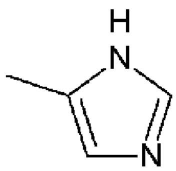

and $R_2$ is -C(O)-$(CH_2)_4$-C(O)-

**2.** A method for in vitro chelating metal ions by means of dicarboxylic acid bisamide derivative of general formula:

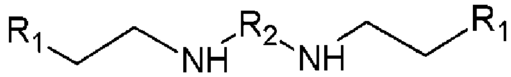,

wherein:

$R_1$ is a group selected from:

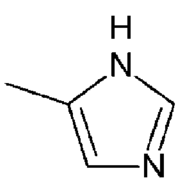, 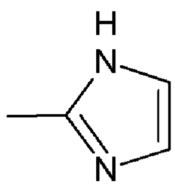, 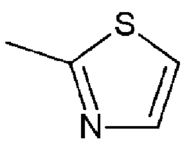, 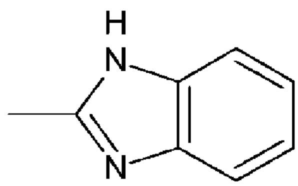,

and

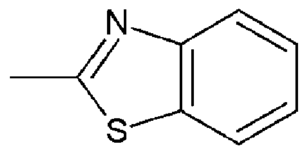;

and

$R_2$ is a group selected from: $-C(O)-(CH_2)_0-C(O)-$, $-C(O)-(CH_2)_1-C(O)-$, $-C(O)-(CH_2)_2-C(O)-$, $-C(O)-(CH_2)_3-C(O)-$, $-C(O)-(CH_2)_4-C(O)-$, $-C(O)-CH_2-CH(CH_3)-CH_2-C(O)-$, and $-C(O)-CH_2-C(CH_3)_2-CH_2-C(O)-$, or a group selected from

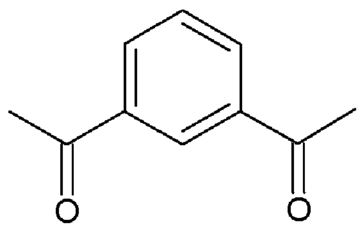, 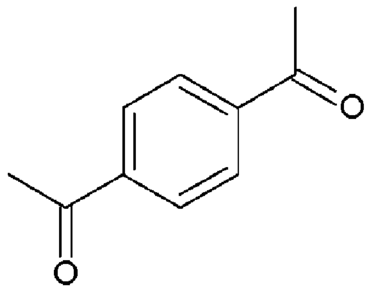, and 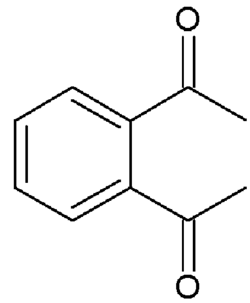

or a pharmaceutically acceptable salt thereof.

**3.** The method of claim 2 wherein the dicarboxylic acid bisamide derivative is able to chelate ions of Zn, Cu, Fe, Mg, and Ca.

**4.** A pharmaceutical composition for use in the prevention and/or treatment of one or more disease selected from a viral disease, a cardiovascular disease, a neurodegenerative disease, a cancer disease, diabetes and its cardiovascular complications, iron-excessive anaemia, porphyria cutanea tarda, alcoholism or alcoholic cirrhosis; or in the treatment of transition metal salt poisoning, or in the treatment of a disease associated with metal-dependent free-radical oxidation reaction, or in the treatment of a disease caused by microbial toxins; wherein said composition comprises an effective amount of a compound of general formula I:

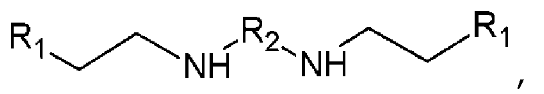,

wherein

$R_1$ is a 5-membered unsaturated heterocyclic group comprising from 1 to 2 heteroatoms selected from N and/or S, optionally condensed with a 6-membered unsaturated cyclic group;

$R_2$ is $-C(O)-R_3-C(O)-$, wherein $R_3$ is $-(CH_2)_n-$ optionally substituted with one to two $C_1$-$C_6$alkyl groups or phenyl; and

n is an integer from 0 to 4;

or a pharmaceutically acceptable salt thereof.

**5.** The pharmaceutical composition for use according to claim 4, wherein the viral disease is caused by hepatitis C virus, human papilloma virus, HIV, or oncogenic RNA virus, such as leukemia virus, or wherein the cardiovascular disease is caused by cytostatic cardiotoxicity, cholesterol deposition, or hypertension.

**6.** The pharmaceutical composition for use according to claim 4, wherein the disease associated with metal-dependent free-radical oxidation reaction is an age-related disease, such as cataract, retinal disease, or skin pigmentation; stroke sequelae; atherosclerosis; or an inflammatory disease, such as osteoarthritis or rheumatoid arthritis.

**7.** The pharmaceutical composition for use according to claim 4, wherein the neurodegenerative disease is Alzheimer's disease, Parkinson's disease, Wilson's disease, Huntington's disease, amyotrophic lateral sclerosis, or a prion disease.

**8.** The pharmaceutical composition for use according to claim 4, wherein the disease caused by microbial toxins is botulism or gaseous gangrene.

**9.** A compound of formula I:

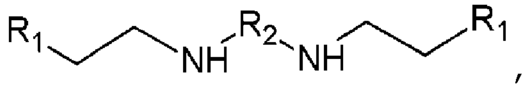

wherein

$R_1$ is a 5-membered unsaturated heterocyclic group comprising from 1 to 2 heteroatoms selected from N and/or S, optionally condensed with a 6-membered unsaturated cyclic group;
$R_2$ is -C(O)-$R_3$-C(O)-, wherein $R_3$ is $-(CH_2)_n-$ optionally substituted with one to two $C_1$-$C_6$alkyl groups or phenyl; and
n is an integer from 0 to 4;
or a pharmaceutically acceptable salt thereof for use in preventing and/or treating a disease selected from a viral disease, a cardiovascular disease, a neurodegenerative disease, a cancer disease, diabetes and its cardiovascular complications, iron-excessive anaemia, porphyria cutanea tarda, alcoholism or alcoholic cirrhosis, transition metal salt poisoning, a disease associated with metal-dependent free-radical oxidation reaction, a disease caused by microbial toxins.

**10.** The compound for use according to claim 9, wherein the viral disease is caused by hepatitis C virus, human papilloma virus, HIV, or oncogenic RNA virus, such as leukemia virus.

**11.** The compound for use according to claim 9, wherein the cardiovascular disease is caused by cytostatic cardiotoxicity, cholesterol deposition, or hypertension.

**12.** The compound for use according to claim 9, wherein the disease associated with metal-dependent free-radical oxidation reaction is an age-related disease, such as cataract, retinal disease, or skin pigmentation; stroke sequelae; atherosclerosis; or an inflammatory disease, such as osteoarthritis, or rheumatoid arthritis.

**13.** The compound for use according to claim 9, wherein the neurodegenerative disease is Alzheimer's disease, Parkinson's disease, Wilson's disease, Huntington's disease, amyotrophic lateral sclerosis, or a prion disease.

**14.** The compound for use according to claim 9, wherein the disease caused by microbial toxins is botulism or gaseous gangrene.

**15.** A method for producing a dicarboxylic acid bisamide derivative of general formula I:

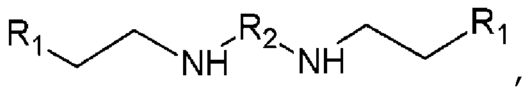

wherein

$R_1$ is a 5-membered unsaturated heterocyclic group comprising from 1 to 2 heteroatoms selected from N and/or S, optionally condensed with a 6-membered unsaturated cyclic group;
$R_2$ is -C(O)-$R_3$-C(O)-, wherein $R_3$ is $-(CH_2)_n-$ optionally substituted with one to two $C_1$-$C_6$alkyl groups or phenyl; and
n is an integer from 0 to 4;
comprising condensing a dicarboxylic acid of general formula II:

$$R_4O\text{-}C(O)\text{-}R_3\text{-}C(O)\text{-}OR_4,$$

wherein $R_3$ is $-(CH_2)_n-$ optionally substituted with one to two $C_1$-$C_6$alkyl groups or phenyl,
n is an integer from 0 to 4, and
$R_4$ is hydrogen or $C_1$-$C_6$alkyl,

with an amine of general formula III:

$NH_2$-$(CH_2)_2$-$R_1$,

wherein $R_1$ is a 5-membered unsaturated heterocyclic group comprising from 1 to 2 heteroatoms selected from N and/or S, optionally condensed with a 6-membered unsaturated cyclic group under heating, and optionally in the presence of a solvent, or in a molar ratio of 1:2-2.5 in a tetrahydrofuran solution in the presence of a condensing agent.

**16.** The method of claim 15, wherein the solvent is selected from a dicarboxylic acid dimethyl or diethyl ether and the step of heating is conducted at a temperature of between 150 and 170°C.

**17.** The method of claim 15, wherein the condensing agent is carbonyldiimidazole.

**Patentansprüche**

**1.** Ein Dicarbonsäurebisamid-Derivat der allgemeinen Formel:

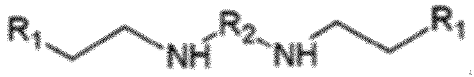,

wobei

$R_1$ eine Gruppe ist, ausgewählt aus:

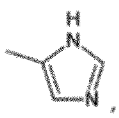, 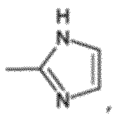, 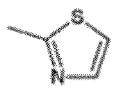, 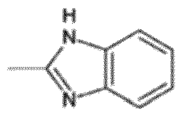,

und

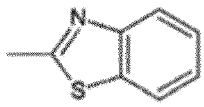;

und
$R_2$ eine Gruppe ist, ausgewählt aus: -C(O)-$(CH_2)_0$-C(O)-, -C(O)-$(CH_2)_1$-C(O)-, -C(O)-$(CH_2)_2$-C(O)-, -C(O)-$(CH_2)_3$-C(O)-, -C(O)-$(CH_2)_4$-C(O)-, -C(O)-$CH_2$-CH($CH_3$)-$CH_2$-C(O)-, und -C(O)-$CH_2$-C$(CH_3)_2$-$CH_2$-C(O)-, oder eine Gruppe ausgewählt aus

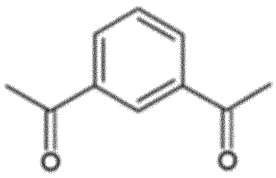, 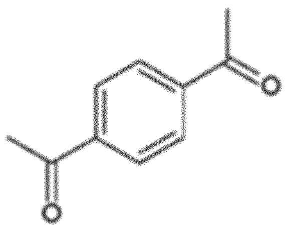, und 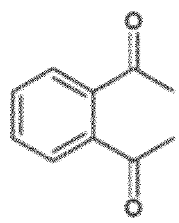

oder ein pharmazeutisch akzeptables Salz davon, mit der Maßgabe, dass die Verbindung keine Verbindung ist, wobei:

$R_1$

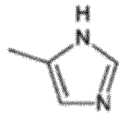

ist und $R_2$ $-C(O)-(CH_2)_3-C(O)-$ ist;
eine Verbindung, wobei:
$R_1$

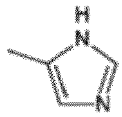

ist und $R_2$ $-C(O)-(CH_2)_4-C(O)-$ ist.

**2.** Ein Verfahren zur in-vitro-Chelatisierung von Metallionen mittels Dicarbonsäurebisamid-Derivats der allgemeinen Formel:

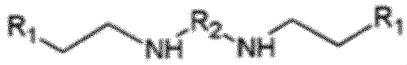,

wobei:

$R_1$ eine Gruppe ist, ausgewählt aus:

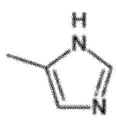, 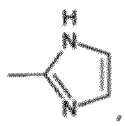, 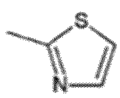, 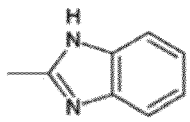,

und

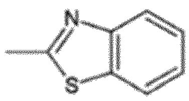;

und
$R_2$ eine Gruppe ist, ausgewählt aus: $-C(O)-(CH_2)_0-C(O)-$, $-C(O)-(CH_2)_1-C(O)-$, $-C(O)-(CH_2)_2-C(O)-$, $-C(O)-(CH_2)_3-C(O)-$, $-C(O)-(CH_2)_4-C(O)-$, $-C(O)-CH_2-CH(CH_3)-CH_2-C(O)-$, und $-C(O)-CH_2-C(CH_3)_2-CH_2-C(O)-$, oder eine Gruppe ausgewählt aus

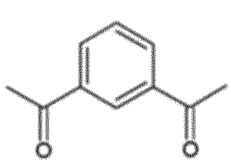, 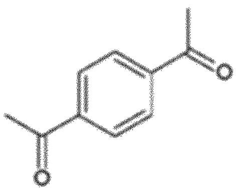, und 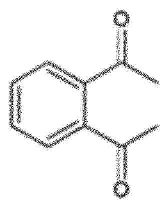

oder ein pharmazeutisch akzeptables Salz davon.

**3.** Das Verfahren nach Anspruch 2, wobei das Dicarbonsäurebisamid-Derivat in der Lage ist, Ionen von Zn, Cu, Fe, Mg und Ca zu chelatisieren.

**4.** Eine pharmazeutische Zusammensetzung zur Verwendung bei der Prävention und/oder Behandlung einer oder mehrerer Erkrankungen, ausgewählt aus einer Viruserkrankung, einer kardiovaskulären Erkrankung, einer neurodegenerativen Erkrankung, einer Krebserkrankung, Diabetes und ihren kardiovaskulären Komplikationen, eisenüberschüssiger Anämie, Porphyria cutanea tarda, Alkoholismus oder alkoholischer Zirrhose; oder bei der Behandlung einer Übergangsmetallsalz-Vergiftung, oder bei der Behandlung einer Erkrankung, assoziiert mit einer metallabhängigen freiradikalischen Oxidationsreaktion, oder bei der Behandlung einer Erkrankung, die durch mikrobielle Toxine verursacht wird; wobei die Zusammensetzung eine wirksame Menge einer Verbindung der allgemeinen Formel I

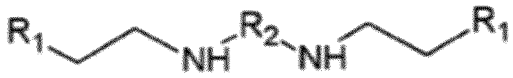

umfasst, wobei

$R_1$ eine 5-gliedrige ungesättigte heterocyclische Gruppe ist, umfassend von 1 bis 2 Heteroatome, ausgewählt aus N und/oder S, optional kondensiert mit einer 6-gliedrigen ungesättigten cyclischen Gruppe;
$R_2$ -C(O)-$R_3$-C(O)- ist, wobei $R_3$ -$(CH_2)_n$- ist, optional substituiert ist mit ein bis zwei $C_1$-$C_6$-Alkylgruppen oder Phenyl; und
n eine ganze Zahl von 0 bis 4 ist;
oder ein pharmazeutisch akzeptables Salz davon.

**5.** Die Pharmazeutische Zusammensetzung zur Verwendung gemäß Anspruch 4, wobei die Viruserkrankung verursacht wird durch Hepatitis-C-Virus, humanes Papillomavirus, HIV oder onkogenes RNA-Virus, wie Leukämievirus, oder wobei die kardiovaskuläre Erkrankung verursacht wird durch zytostatische Kardiotoxizität, Cholesterinablagerung oder Hypertonie.

**6.** Die pharmazeutische Zusammensetzung zur Verwendung gemäß Anspruch 4, wobei die Erkrankung assoziiert mit metallabhängiger freiradikalischer Oxidationsreaktion, eine altersbedingte Erkrankung ist, wie Katarakt, Netzhauterkrankung oder Hautpigmentierung; Schlaganfall-Folgen; Atherosklerose; oder eine entzündliche Erkrankung, wie Osteoarthritis oder rheumatoide Arthritis.

**7.** Die pharmazeutische Zusammensetzung zur Verwendung gemäß Anspruch 4, wobei die neurodegenerative Erkrankung Alzheimer-Krankheit, Parkinson-Krankheit, Wilson-Krankheit, Huntington-Krankheit, amyotrophe Lateralsklerose oder eine Prionenkrankheit ist.

**8.** Die pharmazeutische Zusammensetzung zur Verwendung gemäß Anspruch 4, wobei die durch mikrobielle Toxine verursachte Krankheit Botulismus oder Gasgangrän ist.

**9.** Eine Verbindung der Formel I:

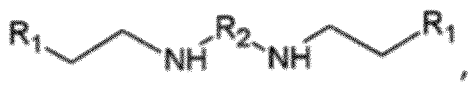

wobei

$R_1$ eine 5-gliedrige ungesättigte heterocyclische Gruppe ist, umfassend von 1 bis 2 Heteroatome, ausgewählt aus N und/oder S, optional kondensiert mit einer 6-gliedrigen ungesättigten cyclischen Gruppe;
$R_2$ -C(O)-$R_3$(O)- ist, wobei $R_3$ -$(CH_2)_n$- ist, optional substituiert mit ein bis zwei $C_1$-$C_6$-Alkylgruppen oder Phenyl; und n eine ganze Zahl von 0 bis 4 ist;
oder ein pharmazeutisch annehmbares Salz davon zur Verwendung bei der Prävention und/oder Behandlung einer Erkrankung, ausgewählt aus einer Viruserkrankung, einer kardiovaskulären Erkrankung, einer neurodegenerativen Erkrankung, einer Krebserkrankung, Diabetes und ihren kardiovaskulären Komplikationen, eisenüberschüssiger Anämie, Porphyria cutanea tarda, Alkoholismus oder alkoholischer Zirrhose, Übergangsmetallsalz-Vergiftung, einer Erkrankung assoziiert mit einer metallabhängigen freiradikalischen Oxidationsreaktion, einer durch mikrobielle Toxine verursachten Erkrankung.

**10.** Die Verbindung zur Verwendung gemäß Anspruch 9, wobei die virale Erkrankung durch Hepatitis-C-Virus, humane Papillomavirus, HIV oder onkogenes RNA-Virus, wie Leukämievirus, verursacht wird.

**11.** Die Verbindung zur Verwendung gemäß Anspruch 9, wobei die kardiovaskuläre Erkrankung durch zytostatische Kardiotoxizität, Cholesterinablagerung oder Hypertonie verursacht wird.

**12.** Die Verbindung zur Verwendung gemäß Anspruch 9, wobei die Erkrankung assoziiert mit metallabhängiger freiradikalischer Oxidationsreaktion, eine altersbedingte Erkrankung ist, wie Katarakt, Netzhauterkrankung oder Hautpigmentierung; Schlaganfallfolgen; Atherosklerose; oder eine entzündliche Erkrankung, wie Osteoarthritis oder rheumatoide Arthritis.

**13.** Die Verbindung zur Verwendung gemäß Anspruch 9, wobei die neurodegenerative Erkrankung Alzheimer-Krankheit, Parkinson-Krankheit, Wilson-Krankheit, Huntington-Krankheit, amyotrophe Lateralsklerose oder eine Prionen-Krankheit ist.

**14.** Die Verbindung zur Verwendung gemäß Anspruch 9, wobei die durch mikrobielle Toxine verursachte Erkrankung Botulismus oder gasförmiges Gangrän ist.

**15.** Ein Verfahren zur Herstellung eines Dicarbonsäurebisamid-Derivats der allgemeinen Formel I:

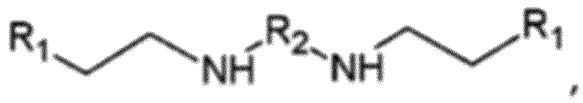

wobei

$R_1$ eine 5-gliedrige ungesättigte heterocyclische Gruppe ist, umfassend von 1 bis 2 Heteroatome, ausgewählt aus N und/oder S, optional kondensiert mit einer 6-gliedrigen ungesättigten cyclischen Gruppe;
$R_2$ -C(O)-$R_3$(O)- ist, wobei $R_3$ -$(CH_2)_n$- ist, optional substituiert mit ein bis zwei $C_1$-$C_6$-Alkylgruppen oder Phenyl; und
n eine ganze Zahl von 0 bis 4 ist;
umfassend Kondensieren einer Dicarbonsäure der allgemeinen Formel II:

$$R_4O\text{-}C(O)\text{-}R_3\text{-}C(O)\text{-}OR_4,$$

wobei $R_3$ -$(CH_2)_n$- ist, optional substituiert mit ein bis zwei C1 - C6-Alkylgruppen oder Phenyl,
n eine ganze Zahl von 0 bis 4 ist, und
$R_4$ ist Wasserstoff oder $C_1$-$C_6$-Alkyl,
mit einem Amin der allgemeinen Formel III:

$$NH_2(CH_2)_2\text{-}R_1,$$

wobei $R_1$ eine 5-gliedrige ungesättigte heterocyclische Gruppe ist, umfassend von 1 bis 2 Heteroatome, ausgewählt aus N und/oder S, optional kondensiert mit einer 6-gliedrigen ungesättigten cyclischen Gruppe unter Erhitzen und optional in Gegenwart eines Lösungsmittels oder in einem molaren Verhältnis von 1:2-2,5 in einer Tetrahydrofuranlösung in Gegenwart eines Kondensationsmittels.

**16.** Das Verfahren nach Anspruch 15, wobei das Lösungsmittel ausgewählt ist aus einem Dicarbonsäuredimethyl oder -diethylether und der Schritt des Erhitzens bei einer Temperatur zwischen 150 und 170 °C durchgeführt wird.

**17.** Das Verfahren nach Anspruch 15, wobei das Kondensationsagens Carbonyldiimidazol ist.

## Revendications

**1.** Dérivé de bisamide d'acide dicarboxylique de formule générale :

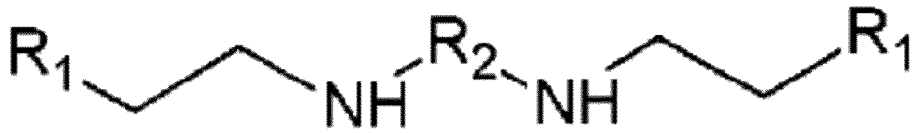,

dans laquelle :

$R_1$ est un groupe sélectionné parmi :

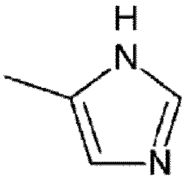, 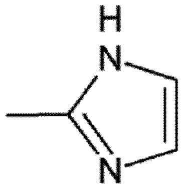, 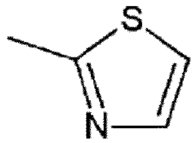, 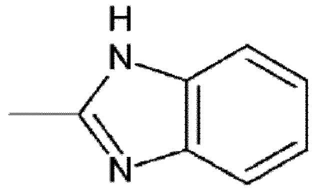,

et

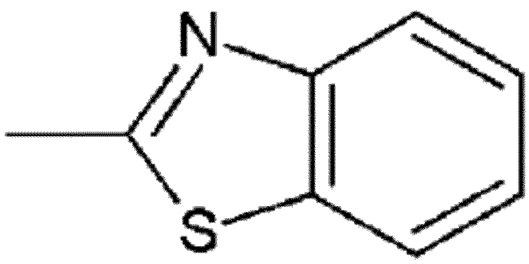;

et

$R_2$ est un groupe sélectionné parmi : $-C(O)-(CH_2)_0-C(O)-$, $-C(O)-(CH_2)_1-C(O)-$, $-C(O)-(CH_2)_2-C(O)-$, $-C(O)-(CH_2)_3-C(O)-$, $-C(O)-(CH_2)_4-C(O)-$, $-C(O)-CH_2-CH(CH_3)-CH_2-C(O)-$, et $-C(O)-CH_2-C(CH_3)_2-CH_2-C(O)-$, ou un groupe sélectionné parmi

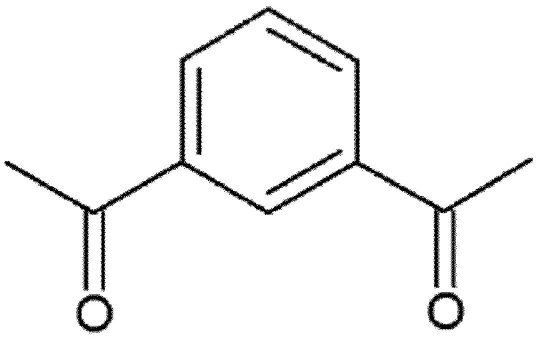, 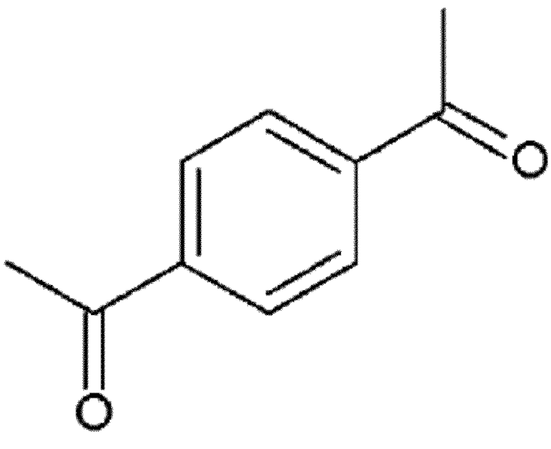,

et

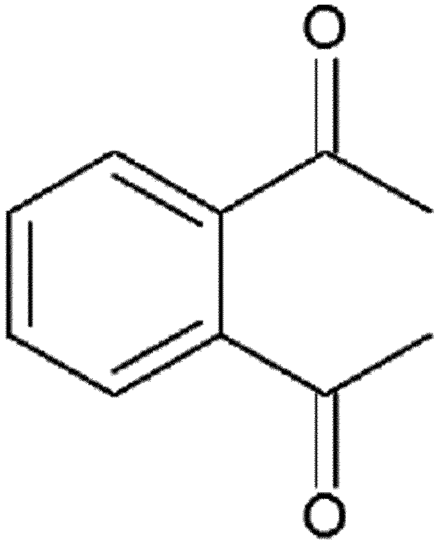

ou sel pharmaceutiquement acceptable de celui-ci, à condition que le composé ne soit pas un composé, dans lequel :

$R_1$ est

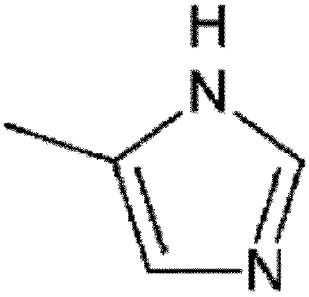

et $R_2$ est -C(O)-$(CH_2)_3$-C(O)- ;
un composé, dans lequel :
$R_1$ est

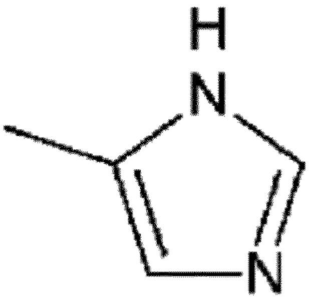

et $R_2$ est -C(O)-$(CH_2)_4$-C(O)-.

**2.** Procédé de chélation in vitro d'ions métalliques au moyen d'un dérivé de bisamide d'acide dicarboxylique de formule générale :

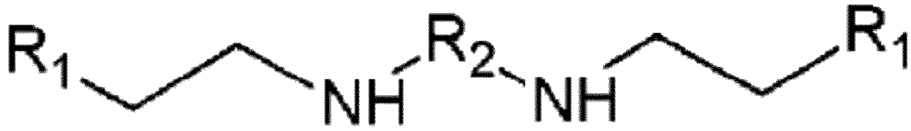,

dans laquelle :

$R_1$ est un groupe sélectionné parmi :

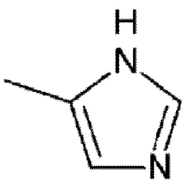, 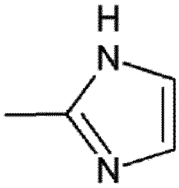, 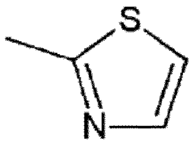, 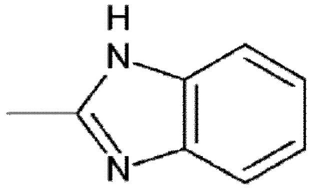,

et

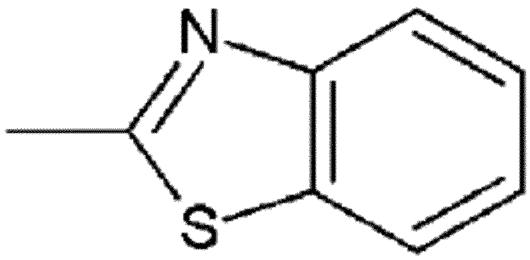;

et

$R_2$ est un groupe sélectionné parmi : $-C(O)-(CH_2)_0-C(O)-$, $-C(O)-(CH_2)_1-C(O)-$, $-C(O)-(CH_2)_2-C(O)-$, $-C(O)-(CH_2)_3-C(O)-$, $-C(O)-(CH_2)_4-C(O)-$, $-C(O)-CH_2-CH(CH_3)-CH_2-C(O)-$, et $-C(O)-CH_2-C(CH_3)_2-CH_2-C(O)-$, ou un groupe sélectionné parmi

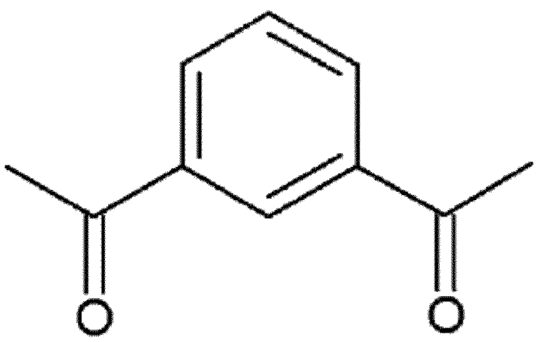, 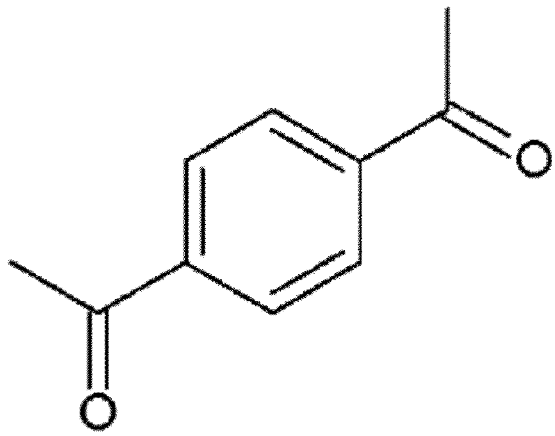,

et

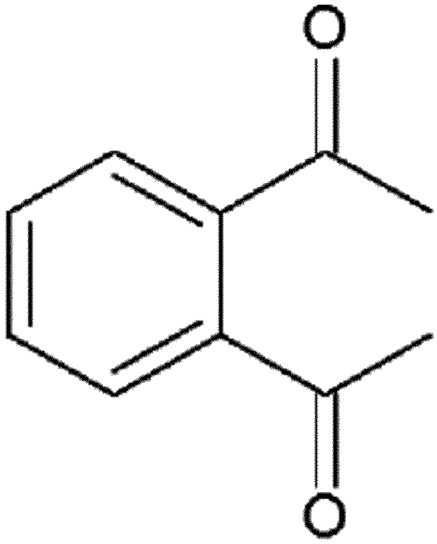

ou d'un sel pharmaceutiquement acceptable de celui-ci.

3. Procédé selon la revendication 2, dans lequel le dérivé de bisamide d'acide dicarboxylique est capable de chélater des ions de Zn, Cu, Fe, Mg et Ca.

4. Composition pharmaceutique destinée à être utilisée dans la prévention et/ou le traitement d'une ou plusieurs maladies sélectionnées parmi une maladie virale, une maladie cardiovasculaire, une maladie neurodégénérative, une maladie cancéreuse, le diabète et ses complications cardiovasculaires, une anémie liée à une surcharge de fer, une porphyrie cutanée tardive, l'alcoolisme ou la cirrhose alcoolique ; ou dans le traitement de l'empoisonnement aux sels de métaux de transition, ou dans le traitement d'une maladie associée à une réaction d'oxydation des radicaux libres dépendant d'un métal, ou dans le traitement d'une maladie provoquée par des toxines microbiennes ; dans laquelle ladite composition comprend une quantité efficace d'un composé de formule générale I :

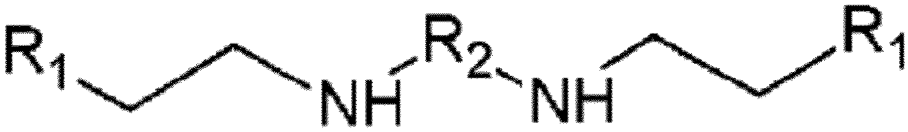

,

dans laquelle

$R_1$ est un groupe hétérocyclique insaturé à 5 membres comprenant 1 à 2 hétéroatomes sélectionnés parmi N et/ou S, condensé en option avec un groupe cyclique insaturé à 6 membres ;
$R_2$ est -C(O)-$R_3$-C(O)-, dans laquelle $R_3$ est $-(CH_2)_n-$ substitué en option par un à deux groupes alkyle en $C_1$-$C_6$ ou phényle ; et
n est un entier de 0 à 4 ;
ou d'un sel pharmaceutiquement acceptable de celui-ci.

5. Composition pharmaceutique destinée à être utilisée selon la revendication 4, dans laquelle la maladie virale est provoquée par le virus de l'hépatite C, le papillomavirus humain, le VIH, ou un virus à ARN oncogène, tel que le virus de la leucémie, ou dans laquelle la maladie cardiovasculaire est provoquée par une cardiotoxicité cytostatique, un dépôt de cholestérol ou l'hypertension.

6. Composition pharmaceutique destinée à être utilisée selon la revendication 4, dans laquelle la maladie associée à une réaction d'oxydation des radicaux libres dépendant d'un métal est une maladie liée à l'âge, telle que la cataracte, une maladie rétinienne ou une pigmentation cutanée ; les séquelles d'un AVC ; l'athérosclérose ; ou une maladie inflammatoire, telle que l'ostéoarthrite ou la polyarthrite rhumatoïde.

7. Composition pharmaceutique destinée à être utilisée selon la revendication 4, dans laquelle la maladie neurodégénérative est la maladie d'Alzheimer, la maladie de Parkinson, la maladie de Wilson, la maladie de Huntington, la sclérose latérale amyotrophique ou une maladie à prion.

**8.** Composition pharmaceutique destinée à être utilisée selon la revendication 4, dans laquelle la maladie provoquée par des toxines microbiennes est le botulisme ou une gangrène gazeuse.

**9.** Composé de formule I :

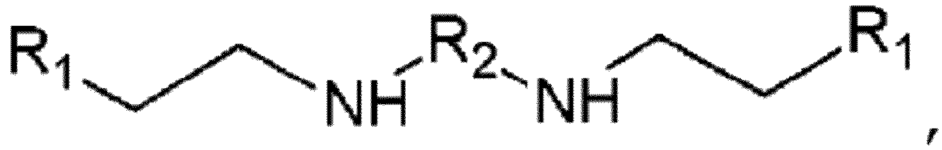

dans laquelle

$R_1$ est un groupe hétérocyclique insaturé à 5 membres comprenant 1 à 2 hétéroatomes sélectionnés parmi N et/ou S, condensé en option avec un groupe cyclique insaturé à 6 membres ;
$R_2$ est $-C(O)-R_3-C(O)-$, dans laquelle $R_3$ est $-(CH_2)_n-$ substitué en option par un à deux groupes alkyle en $C_1$-$C_6$ ou phényle ; et
n est un entier de 0 à 4 ;
ou sel pharmaceutiquement acceptable de celui-ci destiné à être utilisé dans la prévention et/ou le traitement d'une maladie sélectionnée parmi une maladie virale, une maladie cardiovasculaire, une maladie neurodégénérative, une maladie cancéreuse, le diabète et ses complications cardiovasculaires, une anémie liée à une surcharge de fer, une porphyrie cutanée tardive, l'alcoolisme ou la cirrhose alcoolique, l'empoisonnement aux sels de métaux de transition, une maladie associée à une réaction d'oxydation des radicaux libres dépendant d'un métal, une maladie provoquée par des toxines microbiennes.

**10.** Composé destiné à être utilisé selon la revendication 9, dans lequel la maladie virale est provoquée par le virus de l'hépatite C, le papillomavirus humain, le VIH, ou un virus à ARN oncogène, tel que le virus de la leucémie.

**11.** Composé destiné à être utilisé selon la revendication 9, dans lequel la maladie cardiovasculaire est provoquée par une cardiotoxicité cytostatique, un dépôt de cholestérol ou l'hypertension.

**12.** Composé destiné à être utilisé selon la revendication 9, dans lequel la maladie associée à une réaction d'oxydation des radicaux libres dépendant d'un métal est une maladie liée à l'âge, telle que la cataracte, une maladie rétinienne ou une pigmentation cutanée ; les séquelles d'un AVC ; l'athérosclérose ; ou une maladie inflammatoire, telle que l'ostéoarthrite ou la polyarthrite rhumatoïde.

**13.** Composé destiné à être utilisé selon la revendication 9, dans lequel la maladie neurodégénérative est la maladie d'Alzheimer, la maladie de Parkinson, la maladie de Wilson, la maladie de Huntington, la sclérose latérale amyotrophique ou une maladie à prion.

**14.** Composé destiné à être utilisé selon la revendication 9, dans lequel la maladie provoquée par des toxines microbiennes est le botulisme ou une gangrène gazeuse.

**15.** Procédé de production d'un dérivé de bisamide d'acide dicarboxylique de formule générale I :

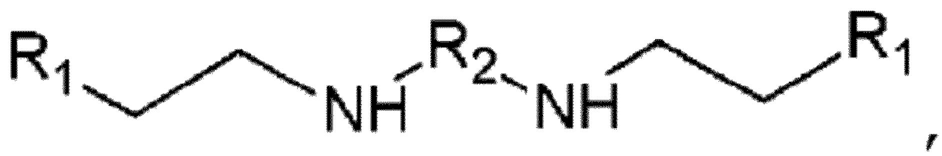

dans laquelle

$R_1$ est un groupe hétérocyclique insaturé à 5 membres comprenant 1 à 2 hétéroatomes sélectionnés parmi N et/ou S, condensé en option avec un groupe cyclique insaturé à 6 membres ;

$R_2$ est -C(O)-$R_3$-C(O)-, dans laquelle $R_3$ est $-(CH_2)_n-$ substitué en option par un à deux groupes alkyle en $C_1$-$C_6$ ou phényle ; et
n est un entier de 0 à 4 ;
comprenant condenser un acide dicarboxylique de formule générale II :

$R_4O$-C(O)-$R_3$-C(O)-$OR_4$,

dans laquelle $R_3$ est $-(CH_2)_n-$ substitué en option par un à deux groupes alkyle en $C_1$-$C_6$ ou phényle,
n est un entier de 0 à 4, et
$R_4$ est hydrogène ou alkyle en $C_1$-$C_6$,
avec une amine de formule générale III :

$NH_2$-$(CH_2)_2$-$R_1$,

dans laquelle $R_1$ est un groupe hétérocyclique insaturé à 5 membres comprenant 1 à 2 hétéroatomes sélectionnés parmi N et/ou S, condensé en option avec un groupe cyclique insaturé à 6 membres en chauffant, et en option en présence d'un solvant, ou dans un rapport molaire de 1/2 à 2,5 dans une solution de tétrahydrofurane en présence d'un agent de condensation.

**16.** Procédé selon la revendication 15, dans lequel le solvant est sélectionné parmi un éther diméthylique ou diéthylique d'acide dicarboxylique et l'étape de chauffage est menée à une température comprise entre 150 et 170°C.

**17.** Procédé selon la revendication 15, dans lequel l'agent de condensation est le carbonyldiimidazole.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZELENIN K.N.** Chelators in medicine. *Soros Educational Journal,* 2001, vol. 7 (1), 45-50
- **ANDREAS J.K.** *Bioorganic & Medicinal chemistry,* 2003, vol. 11, 4599-4613
- **RICE W.G. ; SCHAEFFER C.A. ; HARTEN B.** *Nature,* 1993, vol. 4, 473-475
- **BEERHEIDE W. ; BERNARD H.-U. ; TAN Y.-J. ; GANESAN A.J.** *National Cancer Institute,* 1999, vol. 91 (14), 1211-1220
- **KOZLOV M.V. ; POLYAKOV K.M. ; IVANOV A.V.** *Biochemistry,* 2006, vol. 71 (9), 1253-12594
- **ANDREA URBANI.** *J. Biol. Chem.,* 1998, vol. 30 (273), 18760-18769
- **TIMOTHY L. TELLINGHUISEN ; MATTHEW S. PAULSON ; CHARLES M. RICE.** *J. Virology,* 2006, vol. 80 (15), 7450-7458
- **SPERANDIO D. ; GANGLOFF A.R. ; LITVAK J. GOLDSMITH R.** *Bioorg. Med. Chem. Lett.,* 2002, vol. 12 (21), 3129-3133
- **MARIA BRETNER.** *Acta biochemica polonica,* 2005, vol. 52 (1), 57-70
- **KANEKIYO M. ; ITOH N. ; MANO M.** *Antiviral Res.,* 2000, vol. 47, 207-214
- **YAN CHEN ; CHRISTINE M. LIVINGSTON ; STACY D. CARRINGTON-LAWRENCE.** *J. of Virology,* 2007, vol. 81 (16), 8742-8751
- **MEGAN WHITNALL ; JONATHAN HOWARD ; PREM PONKA.** A class of iron chelators with a wide spectrum of potent antitumor activity that overcomes resistance to chemotherapeutics. *PNAS,* 2006, vol. 103 (40), 14901-14906
- **KIK K. ; SZMIGIERO L.** Dexrazoxane (ICRF-187) - a cardioprotectant and modulator of some anticancer drugs. *Postepy Hig Med Dosw Online,* 2006, vol. 60, 584-590
- **YU YU ; JACKY WONG ; DAVID B. LOVEIOY.** Chelatorsat the cancer coalface: Desferrioxamine to Triapine and Beyond. *Clin. Cancer Res,* 2006, vol. 12, 6876-6883
- **HAIJUN YU ; YUNFENG ZHOU ; STUART E. LIND.** Clioquinol targets zinc to lysosomes in human cancer cells. *Biochem. J.,* 2009, vol. 417, 133-139
- **SHIAN S.G. ; KAO Y.R. ; WU F.Y. ; WU C.W.** Inhibition of invasion and angiogenesis by zinc-chelating agent disulfiram. *Mol. Pharmacol.,* 2003, vol. 64 (5), 1076-84
- **SCHROTEROVA L. ; KAISEROVA H. ; BALIHAROVA V.** The effect of new lipophilic chelators on the activities of cytosolic reductases and P450 cytochromes involved in the metabolism of anthracycline as antibiotics: studies in vitro. *Physiol. Res.,* 2004, vol. 53 (6), 683-691
- **BABIZHAYEV M.A. ; SEGUIN MARIE-C. ; GUEYNEJ J. ; EVSTIGNEEV R.P. ; AGEYEVA E.A. ; ZHELTUCHINA G.A.** L-Carnosine(-alanyl-L-histidine) and carcinine f-alanylhistamine) act as natural antioxidants with hydroxyl-radical-scavenging and lipid-peroxidase activities. *Biochem. J.,* 1994, vol. 304, 509-516
- **ZHOLNIN A.V.** Complex compounds. *Chelyabinsk: ChGMA,* 2000, 28
- **ANNE C. ; BLOMMAERT A.** Thio-derivede disulfides as potent inhibitors of botulinum neurotoxin B: implications of zinc interaction. *Bioorg. Med. Chem.,* 2003, vol. 11 (21), 4655-60
- **BOSSY-WETZEL E. ; SCHWARZENBACHER R. ; LIPTON S.A.** Molecular pathways to neurodegeneration. *Nat. Med,* 2004, vol. 10, 2-9
- **KEVIN J. BARNHAM ; COLIN L. MASTERS ; ASHLEY I. BUSH.** Neurodegenerative diseases and oxidative stress. *Nature Reviews Drug Discovery,* 2004, vol. 3, 205-214
- **YU YU ; JACKY WONG ; DAVID B. LOVEJOY.** Chelators at the Cancer Coalface: Desferrioxamine to Triapine and Beyond. *Clin. Cancer Res.,* 2006, vol. 12, 6876-6883
- **WHITNALL M. ; RICHARDSON D.R.** Iron: a new target for pharmacological intervention in neurodegenerative diseases. *Semin Pediatr Neurol,* 2006, vol. 13, 186-197
- **KEVIN J. BERNHAM ; COLIN L. MASTERS ; ASHLEY I. BUSH.** Neurodegenerative diseases and oxidative stress. *Nature Reviews Drug Discovery,* 2004, vol. 4
- **DANIEL L. COX ; JIANPING PAN ; RAJIV R.P. SINGH.** A Mechanism for Copper Inhibition of Infection Prion Conversion. *Biophysical Journal,* 2006, vol. 91
- **MEGAN WHITNALL.** A class of iron chelators with a wide spectrum of potent antitumor activity that overcomes resistance to chemotherapeutics. *PNAS,* 2006, vol. 103 (40), 14901-14906

- **M.A. PODYMINOGIN ; V.V. VLASSOV.** Synthesis RNA-cleaving molecules mimicking ribonuclease A active center. Design and cleavage of tRNA transcrints. *Nucleic Acids Research,* 1993, vol. 21 (25), 5950-5956
- **ELFRIEDE SCHUHMANN et al.** Bis[platinum(II)] and Bis[Palladium (II)] complexes of α,ω-Dicarboxylic Acid Bis(1,2,4-triaminobutane-N4)-Amides. *Inorg. Chem.,* 1995, vol. 34, 2316-2322
- **SCHUHMANN et al.** Bis[platinum(II)] and Bis[Palladium (II)] complexes of α,ω-Dicarboxylic Acid Bis(1,2,4-triaminobutane-N4)-Amides. *Inorg. Chem.,* 1995, vol. 34, 2316-2322
- **GRINBERG A.A.** An introduction to the chemistry of complex compounds. 1971
- **YU.P. GALAKTIONOV ; M.A. CHISTYAKOV ; V.G. SEVASTIYANOV.** Step Stability Constants of Group IIIB Metal and Lanthanide Complexes with Acetylacetone in Aqueous Solution and the Solubility Products of Metal Triacetylacctonates. *Journal of physical chemistry,* 2004, vol. 78 (9), 1596-1604
- **CLAUDIA A. BLINDAUER ; M. TAHIR RAZI ; SIMON PARSONS ; PETER J. SADLER.** *Polyhedron,* 2006, vol. 25, 513-520